Europäisches Patentamt

**European Patent Office**

Office européen des brevets

(11) Numéro de publication: **0 260 160**
A1

## (12) DEMANDE DE BREVET EUROPEEN

(21) Numéro de dépôt: **87401300.6**

(22) Date de dépôt: **10.06.87**

(51) Int. Cl.⁴: **C 12 N 15/00**
C 12 N 1/18, C 12 N 9/34,
C 12 P 19/20, A 21 D 8/04

(30) Priorité: **10.06.86 FR 8608387**
**13.04.87 FR 8705207**
**13.04.87 FR 8705208**

(43) Date de publication de la demande:
**16.03.88 Bulletin 88/11**

(84) Etats contractants désignés:
**AT BE CH DE ES FR GB GR IT LI LU NL SE**

(71) Demandeur: **TRANSGENE S.A.**
**16, rue Henri Régnault**
**F-92400 Courbevoie (FR)**

(72) Inventeur: **Labat, Nathalie**
**6, rue des Dentelles**
**F-67000 Strasbourg (FR)**

**Nguyen-Juilleret, Martine**
**32, avenue du Général de Gaulle**
**F-67000 Strasbourg (FR)**

**Loison, Gérard**
**19, rue du Faubourg National**
**F-67000 Strasbourg (FR)**

**Lemoine, Yves**
**4, rue des Alisiers**
**F-67100 Strasbourg-Neudorf (FR)**

(74) Mandataire: **Warcoin, Jacques et al**
**Cabinet Régimbeau 26, avenue Kléber**
**F-75116 Paris (FR)**

Le (Les) microorganisme(s) a (ont) été déposé(s) auprès la Collection Nationale de Cultures de Microorganismes de l'Institut Pasteur, Paris sous le(s) numéro(s) 2285 TG 2 AMGC; TGF1 AMG1; 2285 TG2 AMGC - AMY 1.
Le titre de l'invention a été modifié (Directives relatives à l'examen pratiqué à l'OEB, A-III, 7.3)
Le (Les) microorganisme(s) a (ont) été déposé(s) auprès CNCM Institut Pasteur sous le(s) numéro(s) I-568, I-650 et I-649.

(54) **Bloc d'expression d'une amyloglucosidase dans une levure ainsi que les levures le contenant.**

(57) L'invention concerne un bloc d'expression d'une amyloglucosidase dans une levure, caractérisé en ce qu'il comporte :
. le gène de l'amyloglucosidase dépourvu de tout ou partie de ses introns ;
. la séquence d'ADN contenant les signaux assurant la transcription du gène de l'amyloglucosidase par la levure ;
. une séquence codante contenant le signal nécessaire à l'export de l'amyloglucosidase mature dans le milieu extracellulaire.
Plus particulièrement, l'invention concerne des blocs d'expression destinés à l'intégration dans le génome d'une levure industrielle.
La présente invention s'étend à la cotransformation simultanée par des blocs d'expression de l'amyloglucosidase et de l'alphaamylase ainsi qu'à l'application à la fermentation, notamment la fermentation panaire.

EP 0 260 160 A1

## Description

BLOC D'EXPRESSION D'UNE AMYLOGLUCOSIDASE DANS UNE LEVURE, LEVURE TRANSFORMEE ET PROCEDE DE PREPARATION D'ENZYME ET PROCEDE DE FERMENTATION.

La présente inventon décrit des procédés permettant de faire sécréter une glucoamylase par différentes souches de levure du genre Saccharomyces, y comprise les souches industrielles qui n'ont pas la capacité de dégrader des substats carbonés complexes tels que l'amidon ou les dextrines.

L'amidon qui est couramment utilisé comme source de carbone dans la fermentation alcoolique doit être préalablement hydrolysé (soit chimiquement, soit enzymatiquement) dans un procédé séparé pour produire des substrats utilisables pour les levures fermentatives.

Il serait désirable donc de construire par recombinaison génétique des souches industrielles qui soient capables de synthétiser une ou plusieurs enzymes dégradant l'amidon en substrats directement assimilables par ces souches.

Plus particulièrement, le but de la présente invention est de construire par recombinaison génétique une souche responsable de la fermentation brassicole capable de dégrader les dextrines résiduelles du moût afin de produire des bières à faible teneur en sucres.

De même, l'enrichissement du moût en sucres fermentescibles permet de penser également à la production de bières à degré d'alcool élevé.

L'utilisation d'une souche brassicole produisant une enzyme responsable de la dégradation des dextrines au cours de la fermentation alcoolique peut s'avérer être un moyen de simplifier et de rentabiliser les procédés de fabrication.

La fabrication de bières à teneur faible en sucres résiduels peut, notamment, être obtenue en ajoutant à diverses étapes du procédé de fabrication une glucoamylase (Amylo 1,6 glucosidase) d'Aspergillus niger. Cette glycoamylase catalyse la libération de résidus glucose à partir des extrémités non réductrices de l'amidon ou des dextrines.

Cette enzyme joue un grand rôle dans la production de sirops de glucose à partir d'amidon et dans la fabrication d'autres boissons alcoolisées. Cette glucoamylase d'Aspergillus niger est acceptée comme additif dans les industries agroalimentaires.

Donc, en premier lieu, l'objet de la présente invention est l'expression de l'alpha-amyloglucosidase d'Aspergillus niger NCI22343 dans une souche de brasserie.

En second lieu, un des intérêts de l'invention est que les souches de brasserie transformées possèdent l'ADN étranger intégré dans un chromosome de la levure. Lorsque l'ADN étranger est intégré dans le chromosome suivant le procédé décrit plus loin, la propriété de production de l'alpha-amyloglucosidase est stable et la souche ne diffère de la souche de départ que par ce nouveau caractère.

La stabilité de l'information peut être aussi obtenue en construisant une structure d'ADN appelée minichromosome, constitué de trois éléments essentiels : telomère, centromère et origine de réplication.

Un autre objet de la présente invention concerne plus particulièrement un bloc fonctionnel d'ADN permettant la sécrétion de l'amyloglucosidase à partir de la levure, caractérisé par :
. le gène de l'amyloglucosidase dépourvu de tout ou partie de ses introns ;
. une séquence d'ADN comportant les signaux assurant la transcription du gène de l'amyloglucosidase par la levure ;
. une séquence condante contenant le signal nécessaire à l'export de l'amyloglucosidase mature dans le milieu extracellulaire ; le choix s'est porté sur un système propre de sécrétion de la levure, celui de la phéromone alpha (1), d'autres systèmes pourraient être utilisés (par exemple le système de la protéine killer (2) ou celui d'une séquence signal synthétique)
. enfin, le bloc d'expression pourra présenter après le gène de l'amyloglucosidase une séquence de terminaison de levure, par exemple celle du gène de la phosphoglycérate kinase.

De préférence, le gène de l'amyloglucosidase sera dépourvu de la totalité de ses introns (cADN).

Parmi les éléments de la séquence assurant la transcription du gène de l'amyloglucosidase, il faut citer les promoteurs, notamment le promoteur de la PGK.

Ce bloc fonctionnel peut être intégré dans un plasmide à réplication autonome comportant, par exemple, une origine de réplication dans la levure, par example l'origine de réplication du plasmide $2\mu$.

Lorsqu'un tel plasmide est utilisé, il est intéressant qu'il s'agisse d'un plasmide navette susceptible de se répliquer dans une bactérie ; pour ce faire,il pourra comporter des éléments de réplication dans une bactérie, par exemple E. coli et un gène de sélection telle que la résistance à un antibiotique, par exemple Amp$_r$.

De façon générale, le plasmide comportera, de préférence, un gène de sélection effectif dans les levures, par exemple un gène complémentant une auxotrophie tel que ura$_3$+, on bien un gène conférant un phénotype dominant, par exemple la résistance au G418.

Lorsque le bloc d'expression est intégré dans le génome de la souche de levure, l'intégration sera effectuée dans un site non essentiel pour la levure, par exemple le gène MFalpha$_1$.

La constitution d'un vecteur d'intégration est connue et sera décrite en détail dans les exemples.

L'invention concerne également les souches comportant un bloc d'expression selon l'invention, qu'il s'agisse de plasmide ou d'une intégration chromosomique.

Parmi les souches transformées, il faut citer, en particulier, les souches de Saccharomyces, notamment S. cerevisiae et S. uvarum, en particulier les souches industrielles telles que les souches brassicoles.

Enfin, l'invention concerne un procédé de préparation d'amyloglycosidase, notamment d'alpha-amyloglucosidase, caractérisé en ce qu'on fait fermenter sur un milieu de culture approprié une souche selon l'invention et on récupère l'enzyme dans le milieu extracellulaire.

L'invention concerne, enfin, un procédé de dégradation de l'amidon ou des dextrines, caractérisé en ce qu'on fait fermenter un milieu industriel contenant de l'amidon ou des dextrines.

Einfin, le gène de l'alpha-amyloglucosidase utilisé est de préférence celui d'Aspergillus niger.

La présente invention invention concerne également l'introduction du gène et l'expression de l'alpha-amylase de B. licheniformis dans une souche de Saccharomyces produisant de l'alpha-amyloglucosidase.

En effet, l'expression simultanée de l'amyloglucosidase et d'une alpha-amylase conduit à une synergie de ces deux activités enzymatiques dans la dégradation de l'amidon ou des dextrines en résidus glucoses.

Ainsi, à partir des souches industrielles telles que Saccharomyces dans lesquelles l'information génétique pour une alphaamyloglucosidase ainsi que celles pour une endo-alpha-amylase (par exemple de Bacillus licheniformis) sont intégrées ensemble dans les chromosomes de la levure, la production d'éthanol peut être obtenue en utilisant comme source de carbone l'amidon.

La présente invention concerne plus particulièrement l'expression simultanée de l'alpha-amylase et de l'amyloglucosidase dans une souche de levure de brasserie, S. uvarum, polyploïde, en particulier la souche 2285 TG2 AMGC, ainsi que l'utilisation de ces souches pour la préparation de l'alpha-amylase.

Par ailleurs, une application de l'invention concerne la transformation de souches de Saccharomyces, en particulier des souches polyploïdes et prototrophes utilisées dans l'industrie de la panification ; il s'agit, par exemple, de la souche de S. cerevisiae TG1.

L'un des objets de la présente invention est, en effet, la mise en oeuvre de l'alpha-amyloglucosidase, en particulier d'Aspergillus niger, lors de la fermentation panaire car l'alpha-amyloglucosidase qui dégrade l'amidon directement en glucose présente un effet bénéfique sur la fermentation panaire puisque ce sucre est directement assimilable par la levure de boulangerie.

L'adjonction de cette enzyme permet d'améliorer les fabrications en rendant la pâte plus sèche au toucher, donc plus facile à traiter en machine, et surtout d'activer de façon notable l'activité fermentative de la levure boulangère, donc de réduire largement les temps de fermentations.

De plus, elle devrait être source d'innovation en permettant la mise au point de produits de panification relativement sucrés, ce qui est pratiquement impossible sans addition d'enzyme dans un processus de fabrication court.

La présente invention concerne donc, en outre, des procédés permettant de faire sécréter une amyloglucosidase par une souche de boulangerie du genre Saccharomyces cerevisiae.

En particulier, l'invention concerne un procédé de fermentation panaire dans lequel au moins l'une des souches utilisées dans la fermentation est une souche telle que décrite précédemment.

Les exemples ci-après sont destinés à illustrer d'autres caractéristiques et avantages de la présente invention et seront décrits en se référant aux figures sur lesquelles :

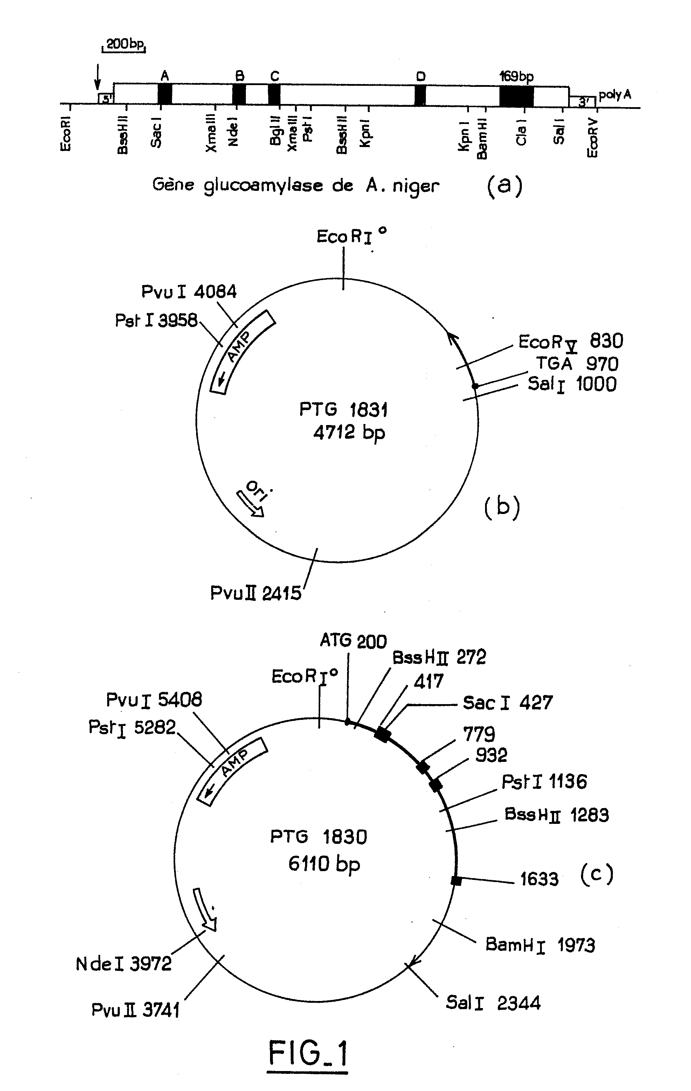

. la figure 1 représente :

a) la structure et la carte de restriction du gène de la glucoamylase d'Aspergillus niger,

b) la structure de plasmide pTG1831,

c) la structure du plasmide pTG1830,

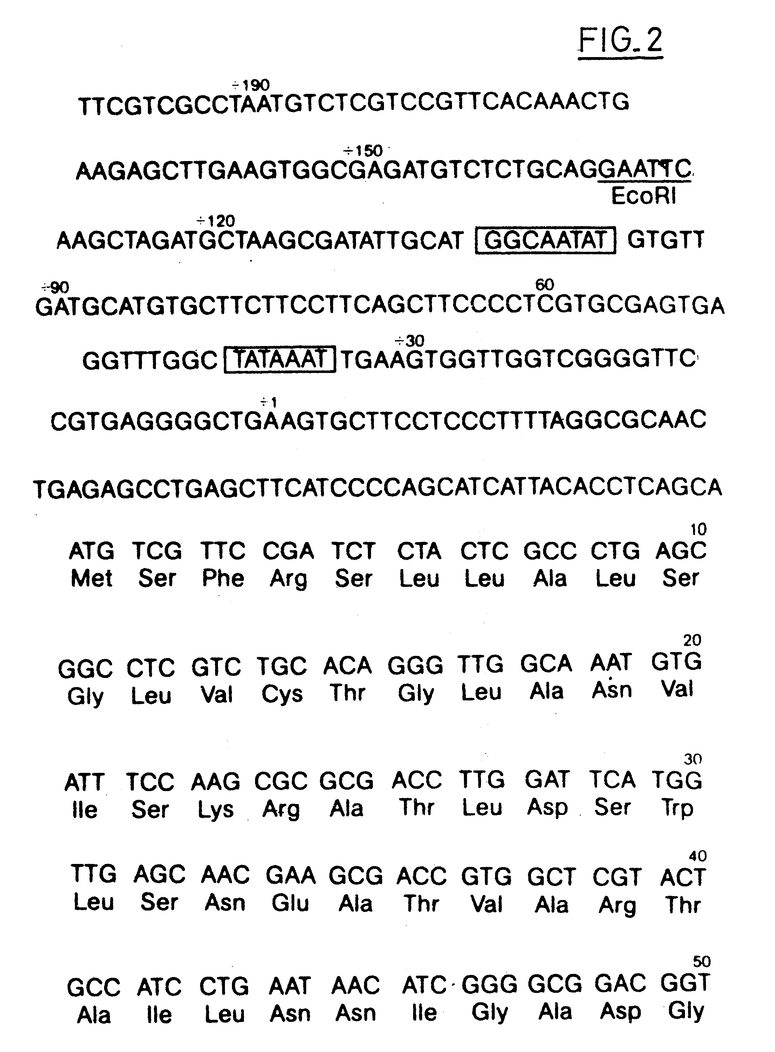

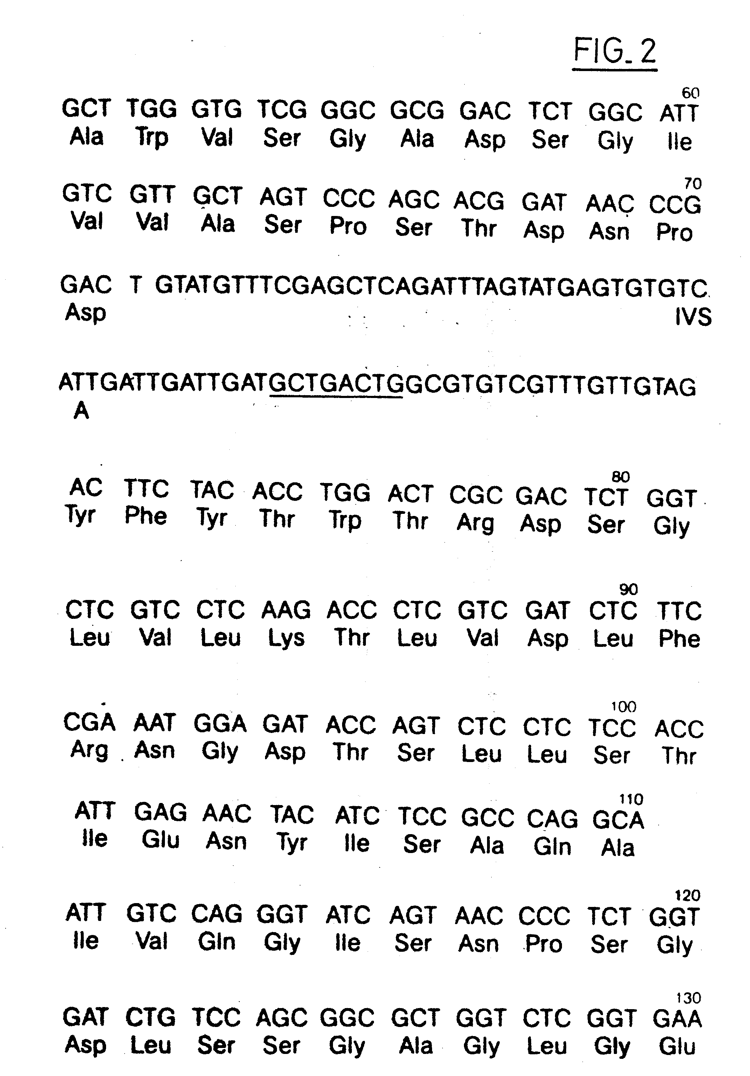

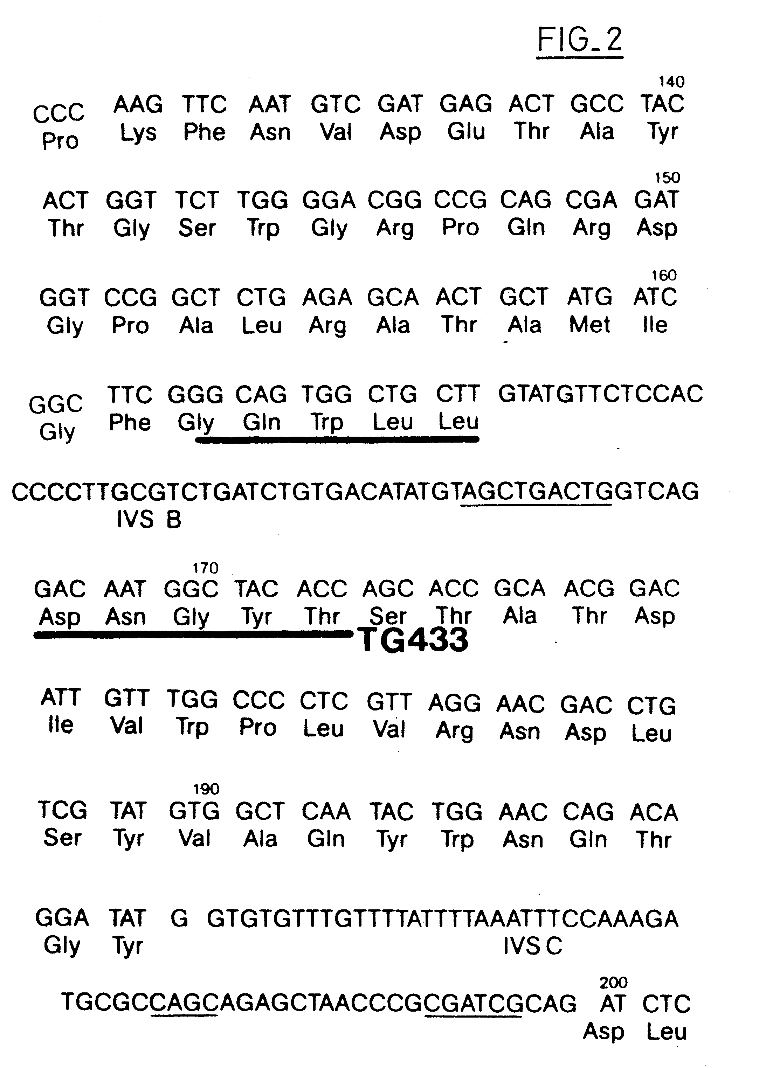

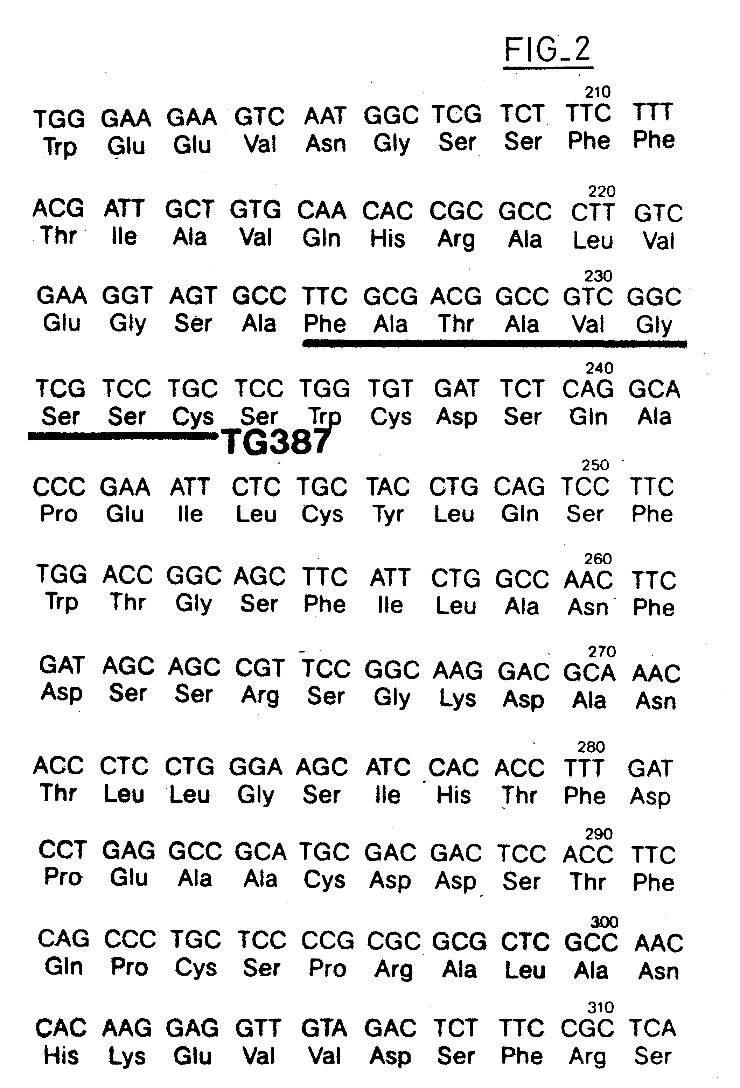

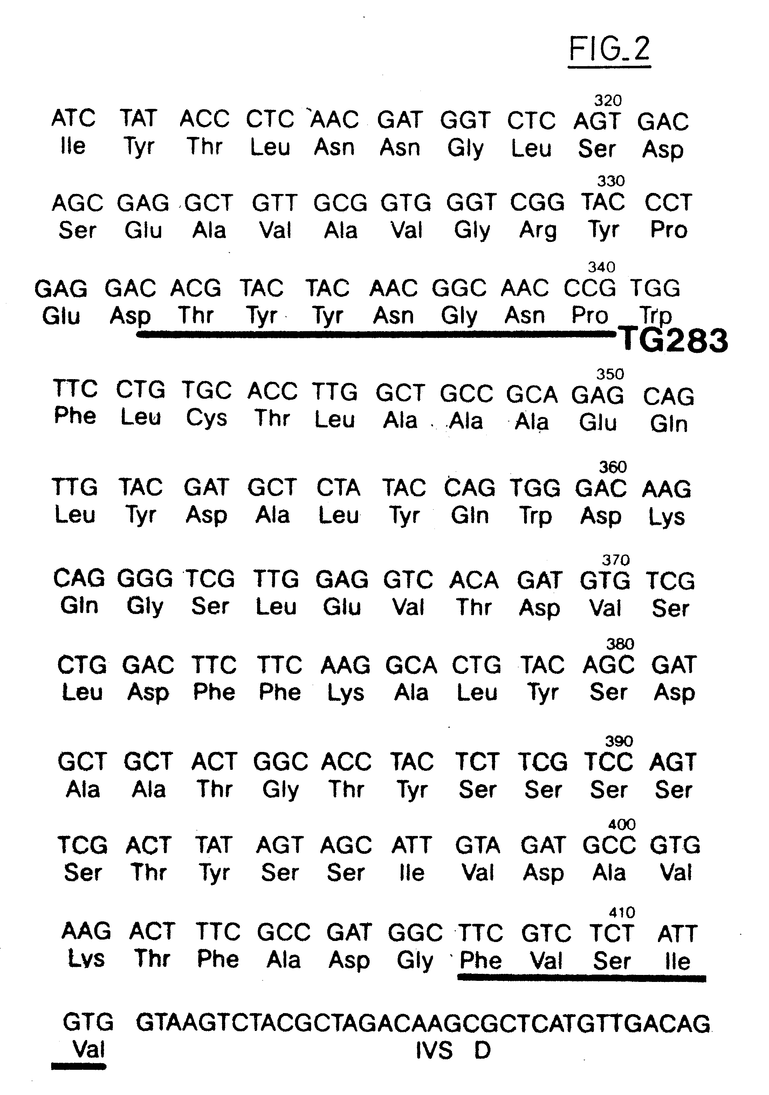

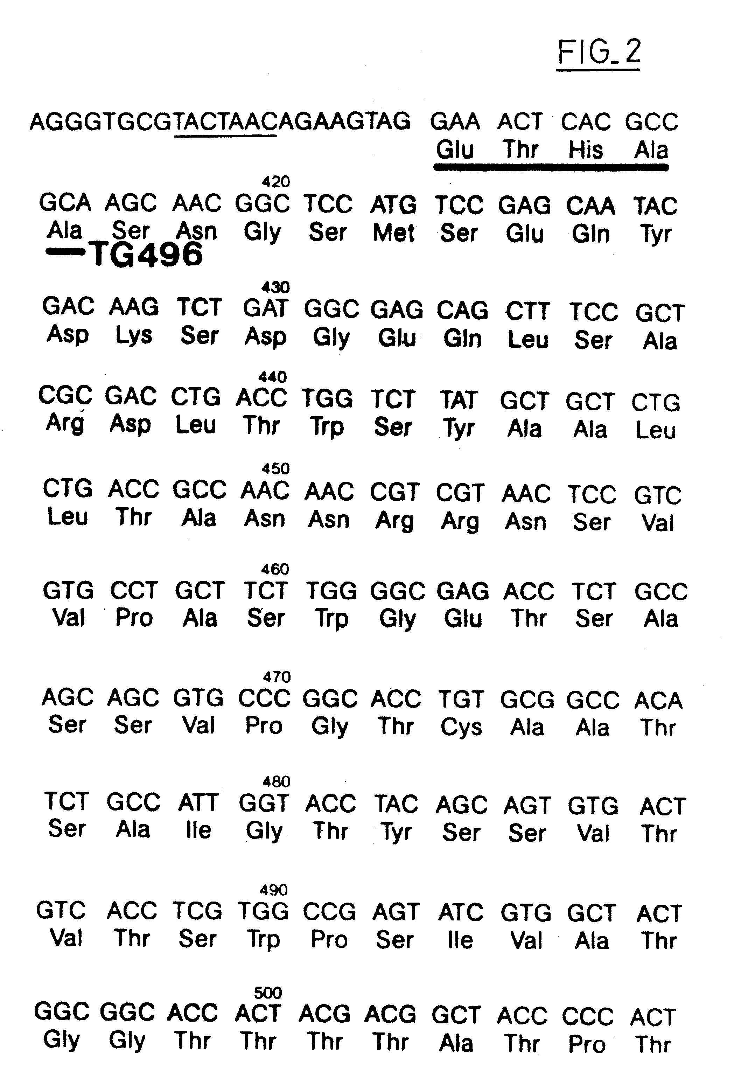

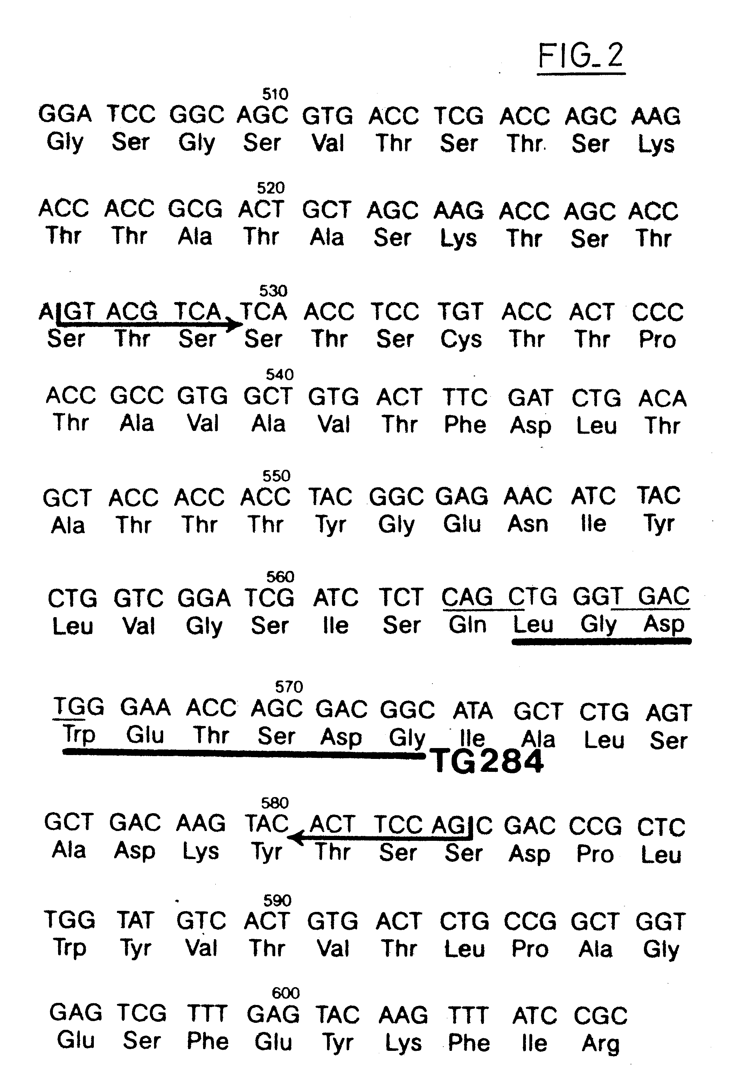

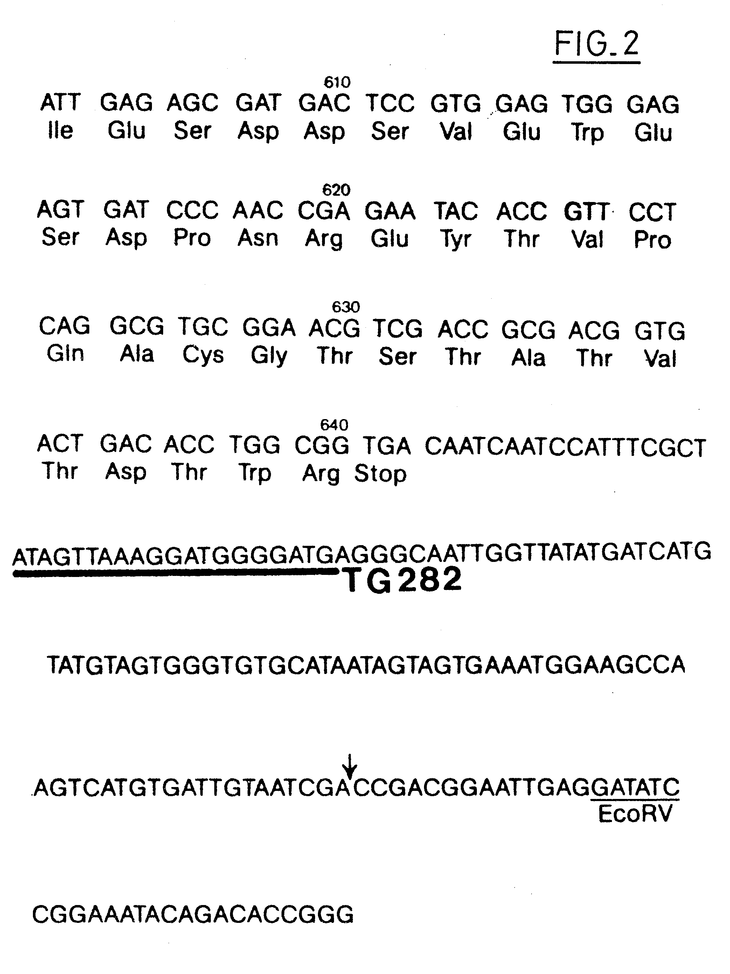

. la figure 2 représente la séquence du gène de l'amyloglucosidase d'Aspergillus niger,

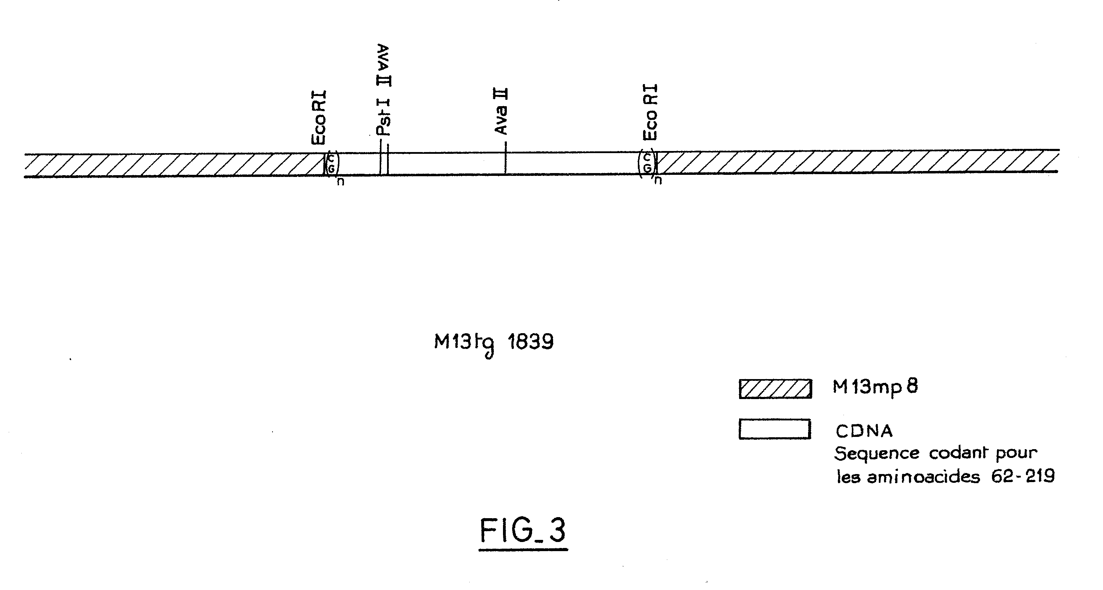

. la figure 3 représente la structure de M13TG1839,

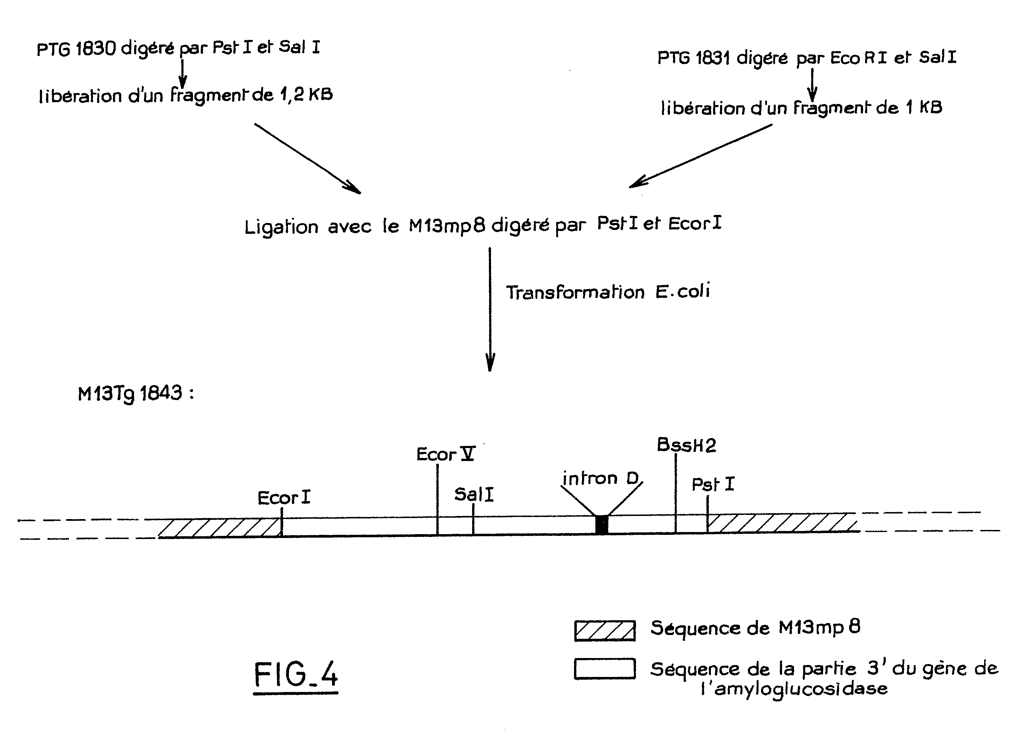

. la figure 4 représente la structure et la construction de M13TG1843,

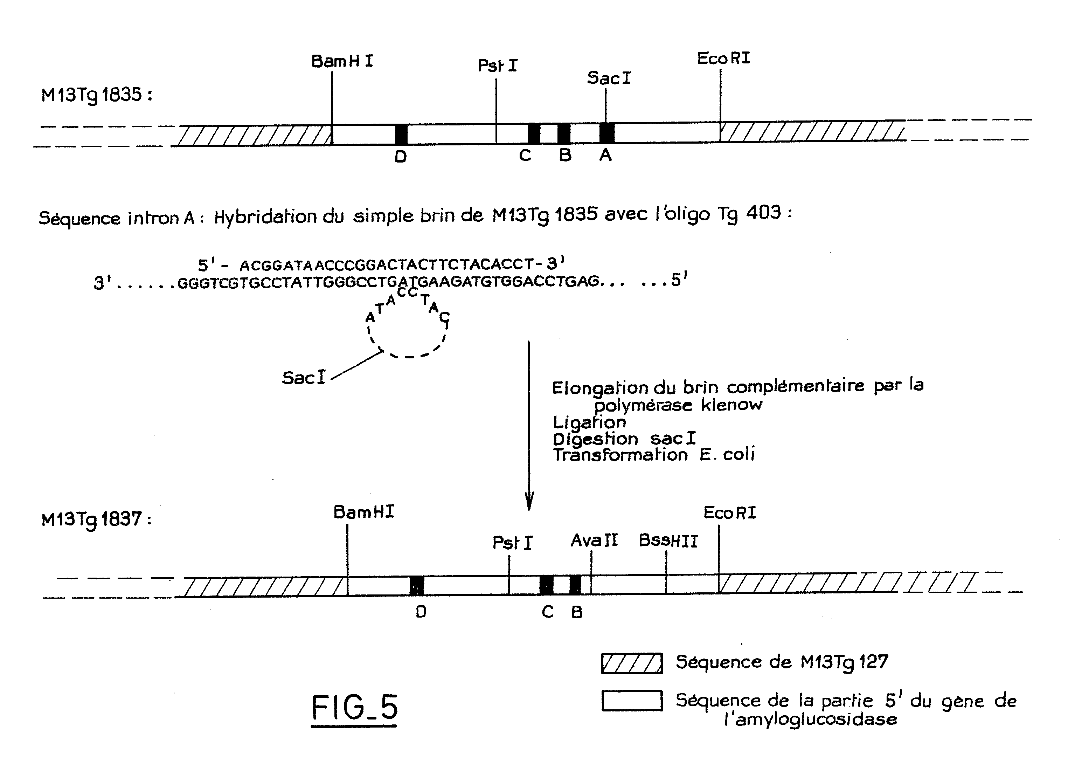

. la figure 5 représente la structure et la construction de M13TG1837,

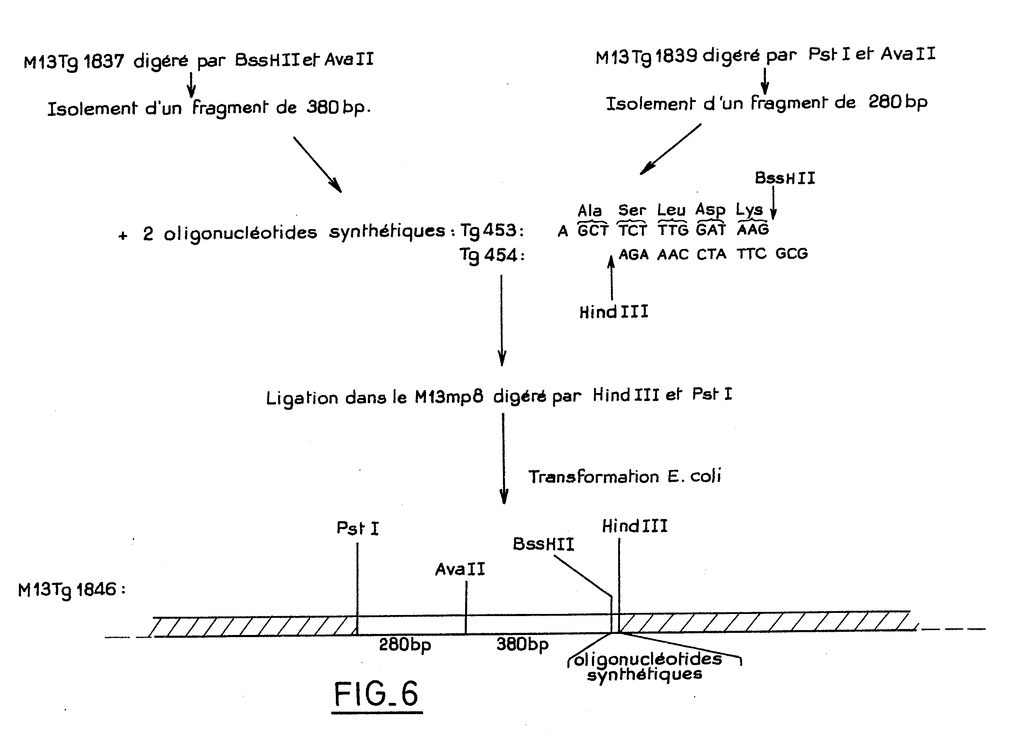

. la figure 6 représente la structure et la construction de M13TG1846,

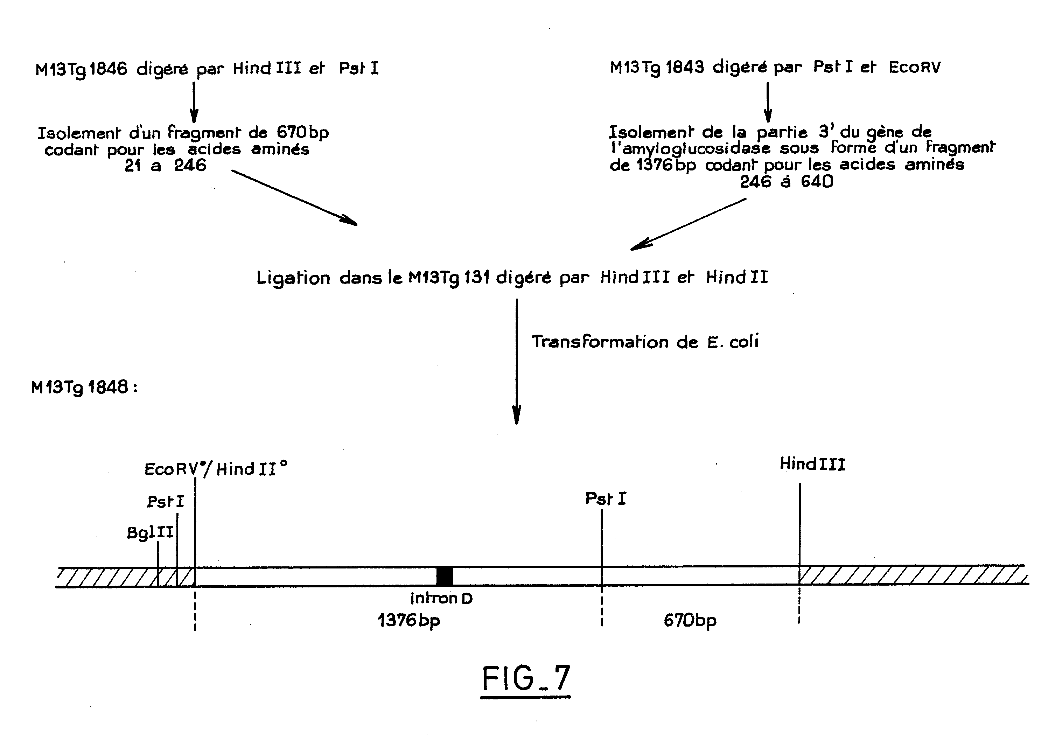

. la figure 7 représente la structure et la construction de M13TG1848,

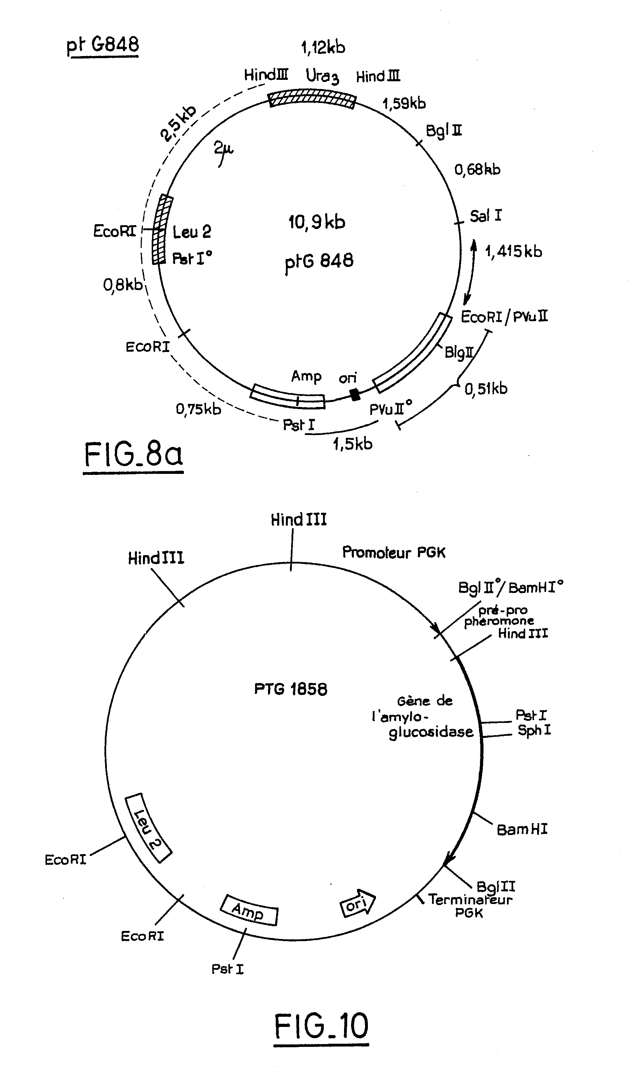

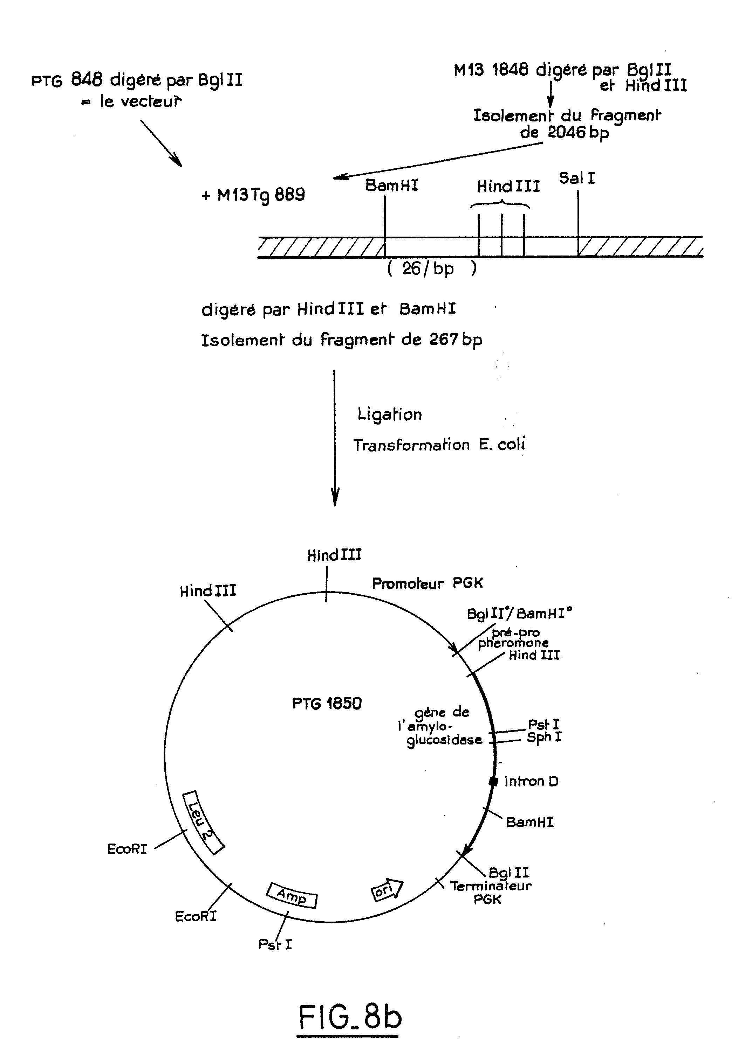

. la figure 8 représente :

a) la structure du plasmide pTG 848,

b) la structure et la construction du plasmide pTG1850,

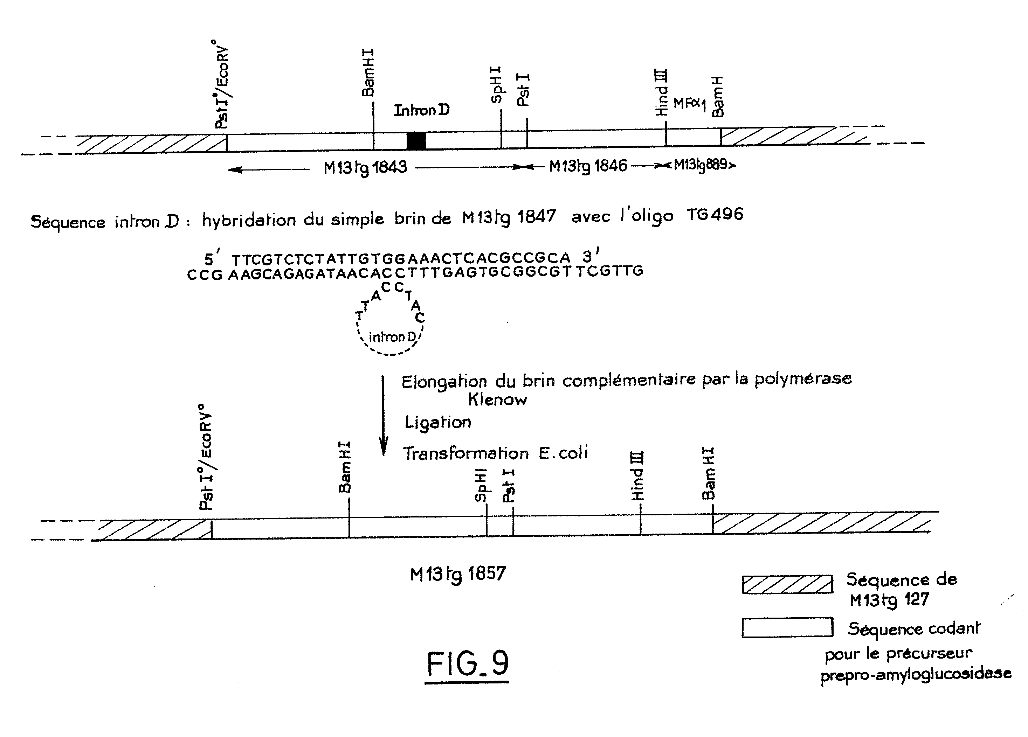

. la figure 9 représente la structure et la construction du M13TG1857,

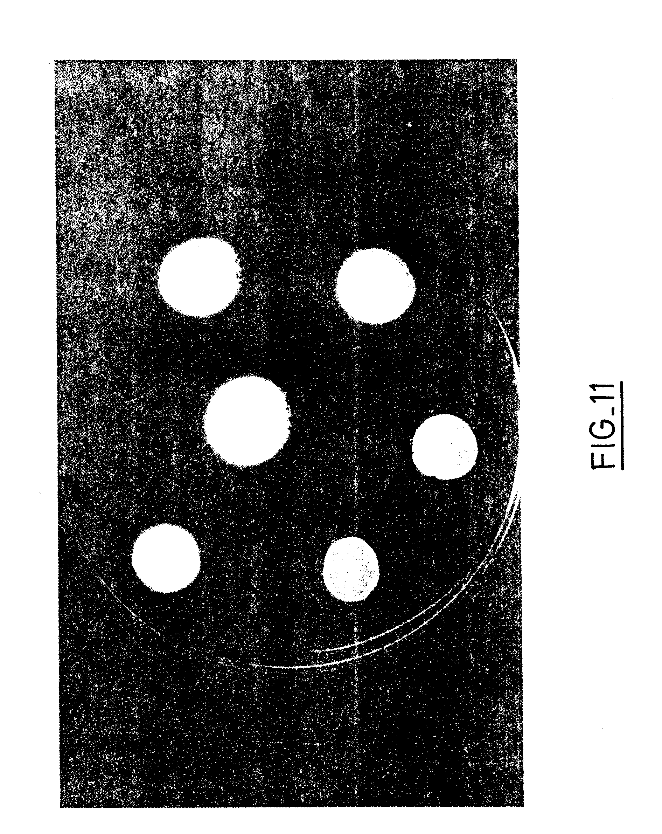

. la figure 10 représente la structure du plasmide pTG1858,

. la figure 11 représente la détection de la production d'amyloglucosidase par la souche TGY2sp13 pTG1858 par test $I_2/KI$,

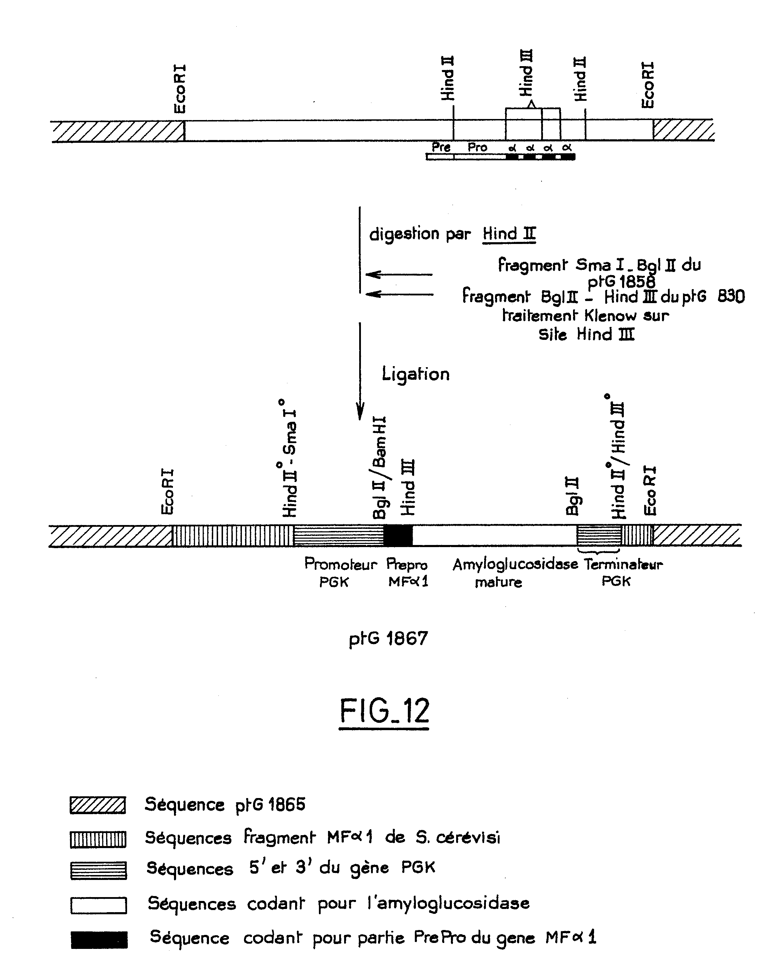

. la figure 12 représente la structure et la construction du plasmide pTG1867,

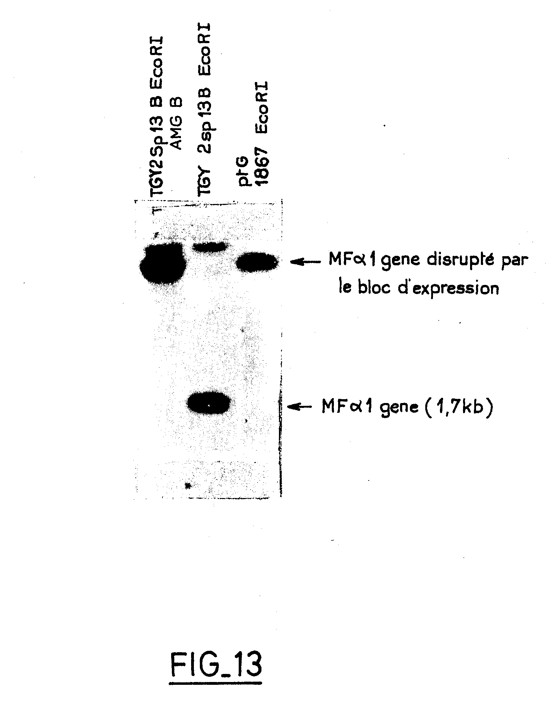

. la figure 13 représente l'hybridation des fragments de restriction EcoRI de l'ADN génomique des souches TGY2sp13b AMGB et TGY2sp13b avec l'oligonucléotide TG529 correspondan à la partie 3′ du gène MFalpha, de S. cerevisiae,

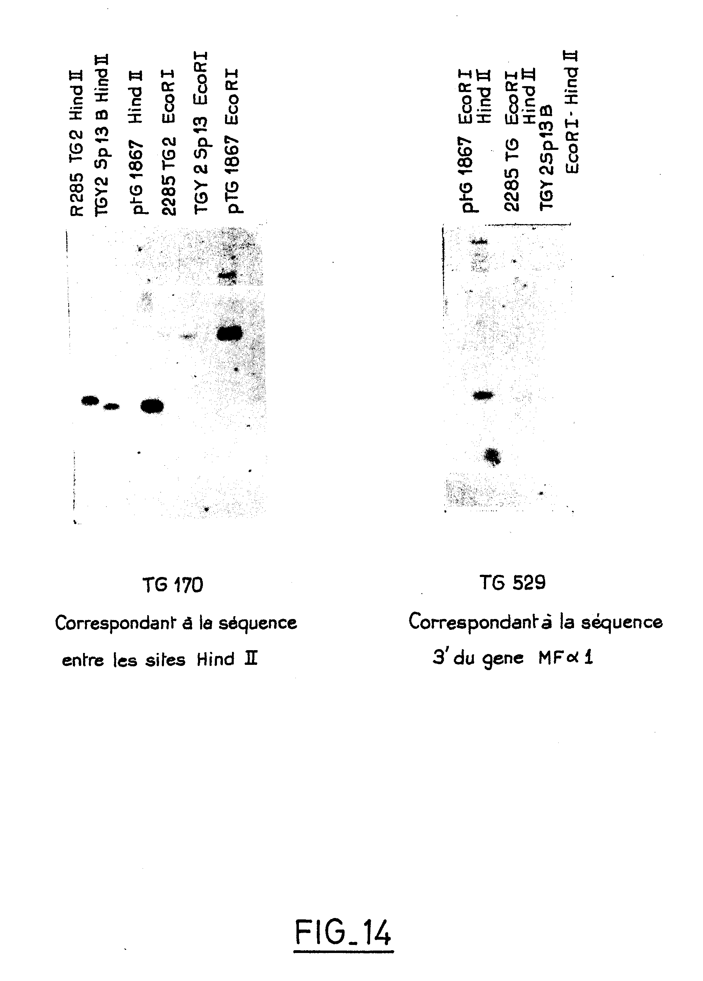

. la figure 14 représente l'hybridation des fragments de restriction de l'ADN génomique des souches TGY2sp13b et 2285TG2 avec soit l'oligonucléotide TG529, soit avec l'oligonucléotide TG170

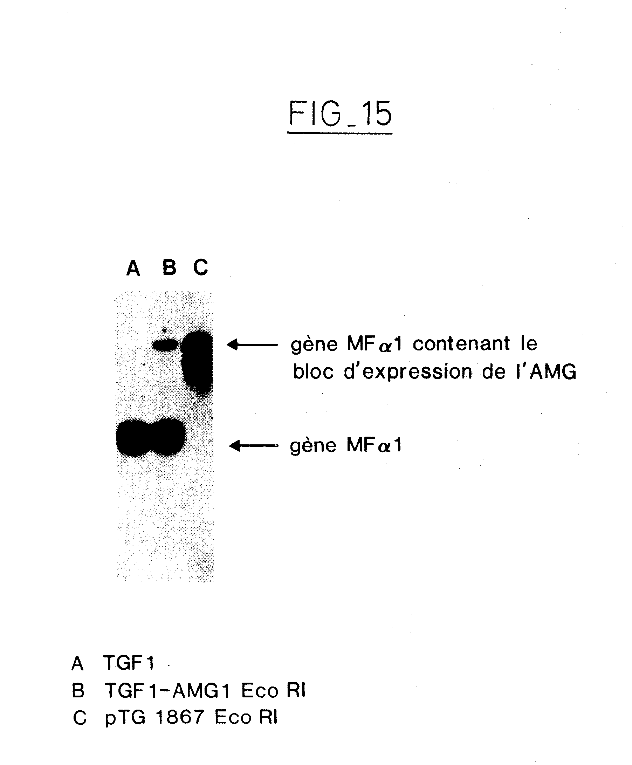

. la figure 15 représente l'hybridation des ADN génomiques digérés par EcoRI des souches TGF1-AMG1 et TGF1 par un oligonucléotide TG529 spécifique de gène MFalphal de Saccharomyces cerevisiae,

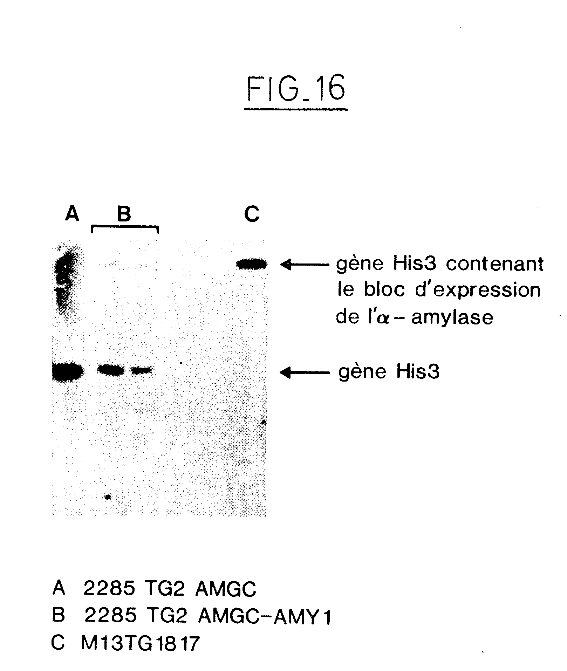

. la figure 16 représente l'hybridation des ADN génomiques digérés par BamHI des souches 2285 TG2 AMGC-AMY1 et 2285 TG2 AMGC par un oligonucléotide TG227 spécifique du gène His3 de S.uvarum.

## EXEMPLE 1

## CLONAGE DU GENE DE L'ALPHA-AMYLOGLUCOSIDASE D'ASPERGILLUS NIGER DANS LE PLASMIDE pBR322

On a complètement digéré par EcoRI et SalI, 1 µg d'ADN génomique d'Aspergillus niger NCI22343 et 0,5 µg d'ADN du plasmide pBR322, puis lié covalemment ces ADN à l'aide de la T4 ADN ligase. On a ensuite transformé la souche Escherichia coli 1106 par les produits de ligation. La sélection des clones portant un insert correspondant au gène de l'alphaamyloglucosidase a été réalisée après transfert des clones recombinants sur feuille de nitrocellulose suivant la méthode classique (3). Les clones recombinants sont été hybridés avec un mélange de trois oligonucléotides TG282, TG283, TG284, définis sur base des séquences déjà publiées (4).

TG282 5′ ATAGTTAAAGGATGGGGATGAGGGC 3′

TG283 5′ AGGACACGTACTACAACGGCAACCC 3′

TG284 5′ CTGGGTGACTGGGAAACCAGCGACG 3′

Deux clones positifs ont été isolés et contiennent un fragment EcoRI-SalI différent. L'un des inserts (2,4 kb) qui hybride deux des trois oligonucléotides (TG283, TG284) correspond à la partie 5′ du gène de l'amyloglucosidase. Par contre, l'autre insert (1 kb) qui hybride avec un seul des trois oligonucléotides (TG282) correspond à la région 3′ du gène de l'amyloglucosidase d'Aspergillus niger (figure 2).

Ces nouveaux plasmides sont appelés pTG1830 et pTG1831 (figure 1).

## EXEMPLE 2

### CLONAGE DU cADN DE L'ALPHA-AMYLOGLUCOSIDASE D'ASPERGILLUS NIGER DANS LE BACTERIOPHAGE LAMBDA

La séquence du gène de l'amyloglucosidase ainsi que celle du cADN de l'amyloglucosidase d'Aspergillus niger (BU-1) a déjà été publiée (4a,b). La comparaison de ces deux séquences révèle la présence de 4 régions introniques dans le gène de l'amyloglucosidase G1 d'Aspergillus niger (BU-1) (figure 2).

A l'exception de l'intron D, qui présente une séquence comparable à la séquence consensus TATAAC,élément indispensable dans le mécanisme d'excision des introns chez la la levure (5), il semble qu'aucun des autres introns ne puisse être excisé par la levure in vivo. Le clonage du cADN de l'amyloglucosidase a donc été entrepris.

On a purifié les ARN messagers d'Aspergillus niger NCI22343 à partir d'une culture de ce champignon sur milieu riche contenant 2 % d'amidon comme source de carbone afin de produire un cADN correspondant à l'amyloglucosidase, par transcription inverse.

Les mARN extraits furent ajoutés à un mélange réactionnel contenant comme amorce de transcription un oligonucléotide dT. La synthèse du cADN a été réalisée selon la méthode décrite par Chang et al. (6). Une banque de cADN fut préparée dans le bactériophage lambda selon Le Bouc et al. (7). L'ADN du bactériophage lambda a été empaqueté in vitro dans les particules phagiques et les phages ainsi formés ont servi à transduire la souche indicatrice Escherichia coli POP101.

La banque de cADN d'Aspergillus niger NCI22343 fut criblée pour l'amyloglucosidase en utilisant comme sonde un oligonucléotide TG433 défini sur base des séquences publiées (4a) :

TG433 5′GGTGTAGCCATTGTCAAGCAGCCACTGCCC 3′

correspondant à la séquence complémentaire de la séquence codant pour les aminoacides 163 à 172 de la protéine mature (figure 2).

Cinq signaux positifs furent détectés. Deux clones sur les cinq furent confirmés par cross hybridation avec un autre oligonucléotide TG387 correspondant à la séquence complémentaire de la séquence codant pour les aminoacides 226 à 236 de la protéine mature (figure 2).

TG387 5′CAGGACGAGCCGACGGCCGTCGCGA 3′

la taille des inserts de ces deux candidats est d'environ 800 pb.

Après analyse par les enzymes de restriction, un des deux fragments a été recloné dans un dérivé M13 (M13mp8) afin de le séquencer. Ce nouveau vecteur est appelé M13TG1839 (figure 3). La séquence du fragment cADN du M13TG1839 codant pour les aminoacides 62 à 319 de la protéine mature s'est avérée identique à celle déjà publiée (figure 2).

## EXEMPLE 3

### CONSTRUCTION D'UN VECTEUR D'EXPRESSION DE L'ALPHA-AMYLOGLUCOSIDASE POUR LA LEVURE S. CEREVISIAE

1) Reconstitution de la partie 3′ du gène de l'amyloglucosidase d'Aspergillus niger

L'ADN du plasmide pTG1830 a été digéré par PstI et SalI de façon à libérer un fragment de 1,2 kb. De même, l'ADN du plasmide pTG1831 à été digéré par EcoRI et SalI libérant un fragment de 1 kb. Ces deux fragments ont été isolés et ligués avec l'ADN du M13mp8 digéré par PstI et EcoRI. On reconstitue un nouveau vecteur appelé M13TG1843 (figure 4). Ce vecteur M13TG1843 possède la partie 3′ du gène de l'amyloglucosidase.

2) Reconstitution de la partie 5′ de la région codante de l'amyloglucosidase mature d'Aspergillus niger

a) Elimination de l'intron A par mutagénèse in vitro

Tout d'abord, le fragment BamHI-EcoRI (2 kb) de pTG1830 contenant la partie 5′ du gène de l'amyloglucosidase a été transféré dans l'ADN phagique M13TG127 entre les sites BamHI et EcoRI.Ceci a donné l'ADN simple brin M13TG1835 à partir duquel la mutagénèse in vitro a été réalisée. Un oligonucléotide TG403 (30 mer) dont la séquence est complémentaire à celles des régions flanquant l'intron A fut employé comme amorce pour la synthèse de l'ADN utilisant comme matrice le simple brin de l'ADN du M13TG1835 (figure 5). La séquence de l'oligonucléotide TG403 est la suivante :

TG403 5′ ACGGATAACCCGGACTACTTCTACACCT 3′

Une picomole d'ADN simple brin du M13TG1835 fut hybridée avec une picomole d'oligonucléotide TG403 dont les extrémités 5′ sont phosphorylées dans un mélange réactionnel qui contient 10 mM Tris pH 7,5, 10 mM $MgCl_2$, 50 mM NaCl. Le mélange fut chauffé à 100°C pendant 3 minutes et incubé pendant 1 heure à température ambiante.

Au mélange d'hybridation, 1,25 µl de chaque déoxynucléotide triphosphate à 5 mM fut ajouté, afin d'obtenir une concentration finale de 500 µM. 5 unités du fragment Klenow de l'ADN polymérase I furent ensuite ajoutées à une unité de $T_4$ ADN ligase ; la réaction d'élongation fut poursuivie à température ambiante pendant deux heures. Après précipitation à l'éthanol, les produits de ligation obtenus furent digérés par l'enzyme de restriction SacI. Ceci permet d'augmenter la fréquence d'obtention des clones recombinants contenant la forme mutée. Les cellules compétentes de la souche E. coli JM103 furent transformées avec le mélange de digestion. On a analysé les plages issues de cette transformation par hybridation ADN-ADN en utilisant comme sonde l'oligonucléotide TG403 décrit précédemment marqué au $^{32}$P par kination suivant les techniques habituelles (8). Plusieurs signaux positifs ont été observés. La séquence d'ADN d'un des clones est identique à celle de l'ADN du M13TG1833 sans la séquence de l'intron A. Ce nouveau phage est appelé M13TG1837 (figure 5).

b) Construction du vecteur M13TG1846

L'ADN du vecteur M13TG1837 a été digéré par BssHII et AvaII. Le fragment BssHII-AvaII de 380 pb contenant la séquence d'ADN codant pour la région $NH_2$-terminale de l'amyloglucosidase mature d'Aspergillus niger (amino-acides 21 à 151) a été isolé (figure 2). De même, le fragment PstI-AvaII de 280 pb du M13TG1839 a été isolé. Ce fragment contient la séquence du cADN codant pour les amino-acides 151 à 256. Ces deux fragments BssHII-AvaII, AvaII-PstI avec deux oligonucléotides synthétiques TG453, TG454 destinés à reconstituer l'extrémité 5′ de l'amyloglucosidase mature et permettant la lecture en phase de la séquence pre-pro de la phéromone alpha suivie de la séquence mature de l'amyloglucosidase (figure 6) sont ligués dans le M13mp8 digéré par HindIII et PstI. Ce nouveau vecteur est appelé M13TG1846 (figure 6).

3) Construction du M13TG1848

Tout d'abord, le fragment HindIII-PstI du M13TG1846 (670 pb) contenant la séquence codant pour les amino-acides 21 à 246 a été isolé. L'ADN du M13TG1843 a été digéré avec PstI et EcoRV et le fragment PstI-EcoRV de 1376 bp issu de cette digestion a été isolé et élué d'un gel. Ce fragment contient la partie 3′ du gène de l'amyloglucosidase (présence de l'intron D) codant pour les amino-acides 246-640. Le site EcoRV est situé en aval du codon stop TGA (environ 140 pb). Le site de polyadénylation est situé à environ 70 pb en amont du site EcoRV. Ces deux fragments sont ligués dans le M13TG131 digéré par HindIII et HindII. Ce nouveau vecteur est appelé M13TG1848 (figure 7). Ce nouveau vecteur a servi comme source de séquence codante d'amyloglucosidase pour le vecteur de sécrétion décrit ci-dessous, malgré la présence du dernier intron D.

4) Construction d'un vecteur d'expression de l'amyloglucosidase pour la levure S. cerevisiae

Le plasmide de départ est le pTG848 (identique au pTG849 décrit dans la demande de brevet français nº 83 15716 à l'exception du gène ura3 dont l'orientation est inverse) ; il est constitué des fragments suivants (figure 8a) :

a) Le fragment EcoRI-HindIII d'environ 3,3 kb, en provenance du plasmide pJDB207 (9). Le site HindIII correspond à la coordonnée 105 du plasmide 2µ forme B, le site EcoRI à la coordonnée 2243. Dans ce fragment le 2µ est de la forme B (10). Ce fragment permet la réplication autonome du plasmide dans la levure.

b) Un fragment HindIII portant le gène URA3 (11) qui permet la sélection du plasmide dans les souches ura3- sur milieu dépourvu de source de pyrimidine.

c) Le grand fragment EcoRI (coordonnée 0)-SalI (coordonnée 650) de pBR322 (12). Dans le site PvuII de ce fragment a été inséré le fragment EcoRI-HindIII dont les extrémités ont été préalablement rendues franches par action de la Klenow en présence des 4 désoxyribonucléotides, de 510 pb et correspondant à la fin du gène PGK (13). L'extrémité EcoRI arasée du gène PGK lorsque jointe à l'extrémité PvuII de pBR322 régénère un site EcoRI.

d) Le fragment HindIII-SalI (2,15 kb) du gène PGK (13).

Le fragment BglII-HindIII du M13TG1848 a été isolé et élué sur gel. Il a été ligué ensuite avec le fragment HindIII-BamHI du M13TG889 contenant la séquence pre-pro de la phéromone alpha dans les sites BglII du plasmide pTG848. On a obtenu un nouveau plasmide appelé pTG1850 (figure 8b).

En résumé, le plasmide pTG1850 possède :

1) un fragment du plasmide 2µ de S. cerevisiae qui permet sa réplication dans Saccharomyces ;

2) le gène URA3+ de S. cerevisiae qui permet la sélection des cellules transformées à partir d'une souche réceptrice ura3- sur milieu dépourvu de source de pyrimidine ;

3) l'origine de réplication du plasmide pBR322 et le gène de résistance à l'ampicilline qui permettent la réplication du plasmide et la sélection des transformés chez E. coli ;

4) le promoteur et le terminateur du gène PGK de levure qui assurent respectivement l'initiation et l'arrêt de transcription du bloc d'expression de l'alpha-amyloglucosidase ;

5) un gène codant pour un précurseur de cette alpha-amyloglucosidase constitué des séquences pre-pro du gène de la phéromone alpha ($MFalpha_1$) de S. cerevisiae fusionné à la séquence codant pour la partie mature de l'alpha-amyloglucosidase qui présente encore son intron D.

5) Transformation d'une souche de laboratoire avec l'ADN du plasmide pTG1850

L'ADN plasmidique pTG1850 fut amplifié dans E. coli, purifié sur un gradient du chlorure de cesium et utilisé pour transformer la souche TGY2sp13b qui est une souche haploïde de S. cerevisiae ayant comme marqueurs auxotrophiques leucine et uracile (Matalpha, ura3-251-373-328, leu2 3-12). Suivant les techniques décrites par Ito et al. (14) les transformants ura+ furent testés pour l'expression du gène de l'amyloglucosidase d'Aspergillus niger. La technique est la suivante : Les clones ura+ ont été repiqués sur milieu solide contenant 2 % d'amidon. Après 48 heures de croissance, 5 ml de mélange aqueux iodé (I$_2$/KI) ont été ajoutés à chaque boîte. L'amidon au contact de l'iode (I$_2$) prend une coloration bleue. Les levures qui produisent de l'amyloglucosidase dans le milieu de culture dégradent l'amidon et il s'ensuit un halo de décoloration autour des colonies. Aucun des transformés ne sécrète de l'amyloglucosidase active.

6) Excision de l'intron D par mutagénèse in vitro

Pour réaliser cette mutagénèse in vitro, on a hybridé un oligonucléotide TG496 dont la séquence est complémentaire de celle des régions flanquant l'intron D de l'ADN du phage M13TG1847 décrit figure 9. Cet oligonucléotide a servi comme amorce pour la synthèse in vitro du brin complémentaire au génome du phage M13TG1847. La séquence de l'oligonucléotide TG496 est la suivante :
TG496 5' TTCGTCTCTATTGTGGAAACTCACGCCG-CA 3'.

La méthode utilisée est identique à celle utilisée pour l'excision de l'intron A. Par contre, aucune enzyme de restriction n'a pu être utilisée pour "saboter" les formes non mutées. Plusieurs signaux positifs ont été observés. L'ADN d'un des clones positifs a été séquencé. L'intron D a été correctement délété. Ce nouveau phage est appelé M13TG1857 (figure 9).

7) Construction du plasmide pTG1858

Tout d'abord, le fragment BamHI-SphI de 670 pb du M13TG1857 a été isolé et purifié sur gel. Il a été ligué avec le grand fragment BamHI-SphI pTG1850. On reconstitue un nouveau vecteur d'expression amyloglucosidase appelé pTG1858. Ce vecteur possède la région codante pour l'amyloglucosidase mature d'Aspergillus niger (figure 10).

EXEMPLE 4

TRANSFORMATION D'UNE SOUCHE DE LABORATOIRE AVEC L'ADN DE pTG1858

L'ADN de pTG1858 a servi à transformer une levure TGY2sp13b (Matalpha, ura3-251-373-328, leu 2-3-18) pour ura+ suivant les techniques décrites (14), la présence d'alphaglucoamylase a été testée autour des colonies comme cela a été décrit ci-dessus (figure 11). Comme attendu, on a trouvé que tous les transformés avec le plasmide de pTG1858 sécrètent de l'amyloglucosidase active. De plus, la croissance des souches de levure transformées par le plasmide pTG1858 fut comparée à celle de la même souche transformée par le plasmide pTG848. Les levures transformées avec les deux plasmides croissent sur milieu minimum contenant 2 % d'amidon comme source de carbone. Cependant la souche transformée par le plasmide pTG1858 montre toujours une meilleure croissance que la souche transformée par le plasmide pTG848 sur ce milieu.

EXEMPLE 5

CARACTERISATION D'UNE ACTIVITE AMYLOGLUCOSIDASE DANS LES SURNAGEANTS DE CULTURE DE LA LEVURE

La quantité d'amyloglucosidase sécrétée par la souche de levure TGY2sp13b contenant le plasmide pTG1858 fut dosée. Après deux jours d'incubation dans le milieu YNBG + 0,5 % casamino acides à 30°C sous une agitation de 250 RPM, la culture de TGY2sp13b contenant pTG1858 a consommé tout le glucose et fut en phase stationnaire ; la culture fut arrêtée à une densité cellulaire approximative de 2,6 x 10$^8$ cellules/ml. La mesure de la quantité d'amylo glucosidase présente dans le surnageant non concentré indique que 360 unités par litre de surnageant d'amyloglucosidase active furent produites (une unité d'activité amyloglucosidase correspond au relargage d'une µmole de glucose/mn à partir de 2 % d'amidon Zulkowsky dans 50 mM tampon acétate de sodium pH 4,3 à 55°C).

EXEMPLE 6

CONSTRUCTION D'UN PLASMIDE D'INTEGRATION POUR SACCHAROMYCES CEREVISIAE

Pour ce faire, on a cloné dans le site EcoRI d'une dérivé du pUC8 (pTG1864) un fragment EcoRI (1,7 kb) correspondant à la région chromosomique portant le gène MFalpha1 de S. cerevisiae (1). Ce nouveau vecteur est appelé pTG1865 (figure 12).

L'ADN de pTG1865 a été digéré par HindII libérant un petit fragment d'environ 0,5 kb interne au gène MFalpha1. On a inséré aux sites HindII du pTG1865 par "Blunt end" ligation le fragment SmaI-BglII du pTG1858 qui comprend la séquence du précurseur pre-pro amyloglucosidase sous contrôle du promoteur du gène de la phosphoglycérate kinase et le fragment BglII-HindIII du pTG830 qui comprend la séquence du terminateur du gène PGK après action de la polymérase Klenow sur le site HindIII. Ce nouveau vecteur est appelé pTG1867 (figure 12). La digestion de pTG1867 par EcoRI libère un fragment d'ADN de levure (4 kb) dans lequel on a intégré le bloc d'expression de l'amyloglucosidase.

EXEMPLE 7

COTRANSFORMATION DE TGY2sp13b EN VUE D'OBTENIR DES LEVURES SACCHAROMYCES AYANT INTEGRE LE BLOC D'EXPRESSION AMYLOGLUCOSIDASE

La présence aux extrémités d'un fragment d'ADN linéaire de régions homologues à une région du génome de levure favorise les évènements de recombinaison entre la région chromosomique et le fragment linéaire. Ceci nous permet de substituer à la séquence chromosomique celle du fragment linéaire qui peut renfermer des séquences étrangères. On peut donc obtenir aussi l'intégration de séquences étrangères dans le chromosome de la levure.

On a donc cotransformé la souche TGY2sp13b (Matalpha ura3-251-373-328, leu 2-3-12) par la technique habituelle (14) avec 2 μg d'ADN de pFL1 (15) conférant le phénotype ura+ et en même temps avec 10 μg d'ADN de pUC1867 préalablement coupé par EcoRI. Sur les 300 clones ura+ obtenus, on a testé ceux qui étaient devenus MFalpha₁. En effet, la substitution au locus MFalpha₁ par le fragment d'ADN linéaire entraîne une inactivation du gène MFalpha₁, c'est-à-dire une perte de production du facteur alpha d'au moins 75 % (16). Le phénotype MFalpha₁ est testé de la façon suivante : Un tapis de cellules FL 100 Mata (5 x 10⁴ cellules) est étalé sur boîte YNBG + 0,5 % casamino acides. On a déposé sur ce tapis en pâté de cellules provenant des clones ura+. Au bout de 48 heures, à température ambiante, on a observé la présence d'un halo d'inhibition de croissance de la souche Mata autour des souches ayant le phénotype MFalpha₁. Ceci traduit le fait que la souche MFalpha₁ sécrète suffisamment de phéromone alpha pour inhiber la croissance des souches Mata. Autour des souches mfalpha₁ aucun halo d'inhibition de croissance n'a été observé. Sur les 300 clones ura+, on a obtenu un transformé mfalpha₁ appelé TGY2sp13bØ AMGB. Ce clone produit un halo de décoloration au test I₂/KI au bout de 5 jours de croissance à 30°C. Donc ce clone TGY2sp13B Ø AMGB produit de l'amylo glucosidase active.

Ce clone exprime l'alpha-amyloglucosidase de façon stable au cours des générations. L'analyse de l'ADN génomique de la souche TGY2sp13b Ø AMGB par Southern révèle que le bloc d'expression amyloglucosidase est intégré dans le génome au locus MFalpha₁ (figure 13) et que l'intégration de ce fragment étranger à été réalisée par un processus d'échange sans intégration de séquence du vecteur pTG1864.

La quantité d'amyloglucosidase sécrétée par la souche de levure TGY2sp13b Ø AMGB et la souche TGY2sp13b contenant le plasmide pTG1858 a été dosée.

Après 2 heures d'incubation à 30°C sous agitation de 250 RPM, la souche TGY2sp13b Ø AMGB produit environ 6 à 8 fois moins d'amyloglucosidase active que la souche TGY2sp13b contenant le plasmide pTG1858.

EXEMPLE 8

INTEGRATION DANS UNE SOUCHE POLYPLOIDE DE BRASSERIE SACCHAROMYCES UVARUM (2285TG2)

Le lieu d'intégration du bloc d'expression de l'amyloglucosidase dans le génome de la souche de brasserie doit être choisi de manière à ne pas modifier la physiologie ni les caractéristiques de la souche 2285TG2. Le choix du site d'intégration s'est porté sur le gène MFalpha₁ responsable de la synthèse du facteur alpha.Une souche de type sexuel Mata ne présente pas d'inhibition de croissance détectable lorsque mise au contact d'une souche de brasserie polyploïde. Le facteur alpha joue un rôle dans la conjugaison entre deux levures de type sexuel opposé. Dans la souche 2285TG2, l'inactivation du gène MFalpha₁ ne doit pas modifier la physiologie de la souche. On a déterminé la présence de ce gène dans la souche 2285TG2 en analysant l'ADN génomique de cette souche par Southern (figure 14). Le gène MFalpha₁ présent dans la souche 2285TG2 apparaît largement homologue à celui de la souche S. cerevisiae. Une seule différence a été décelée au niveau de la région codant pour le facteur alpha situé entre les deux sites HindII (figure 12). Cette zone contient, suivant les souches, un nombre variable de copies de ce facteur alpha (17). La différence observée est vraisemblablement due à la présence au moins d'une copie supplémentaire dans la souche Saccharomyces uvarum. De ce fait, le bloc d'expression de l'amyloglucosidase a été inséré dans les sites HindII du gène MFalpha₁ de S. cerevisiae délimitant la région codant pour le facteur alpha.

La souche 2285TG2 polyploïde et n'ayant donc aucun marqueur d'auxotrophie, la cotransformation avec le pTG1867 se fait en utilisant un plasmide conférant un phénotype dominant. On a utilisé la résistance au G418. Ce plasmide que l'on a utilisé est un dérivé du plasmide pTG 1861 décrit dans le brevet français n° 84 04453. Le plasmide pTG1825 ne diffère de pTG1861 que par l'absence d'un ATG hors phase qui a été délété par mutagénèse in vitro pour permettre une meilleure expression du gène de résistance au G418.

On a transformé la souche 228TG2 avec 1 μg d'ADN de pTG1825 et 10μg de pTG1867 préalable-ment digéré par EcoRI par la technique dérivé de Dickson (18). La modification suivante a été réalisée : environ 15 heures après la transformation, on a ajouté une surcouche sur milieu complet (10 ml) contenant de la généticine, G418 (Difco) de façon à ce que la concentration finale de l'antibiotique soit de 300 μg/ml dans la boîte.

Sur 300 clones G418 résistants, un clone 2285TG2 AMGC produit de l'amyloglucosidase. Ce clone exprime l'alpha-amyloglucosidase de façon stable au cours des générations. L'analyse de l'ADN génomique de la souche 2285TG2 AMGC par Southern révèle que le bloc d'expression amyloglucosidase est intégré dans le génome au locus MFalpha₁. La quantité d'amyloglucosidase sécrétée par la souche brassicole a été dosée. Après 3 jours d'incubation en milieu complet YPG à 30°C sous agitation à 250 RPM, la culture de 2285 TG2 AMGC produit à une densité cellulaire approximative de 4 x 10⁷ cellules/ml, 100 U par litre de surnageant d'alpha-amyloglucosidase active.

EXEMPLE 9

La même expérience que celle décrite à l'exemple 8 est réalisée au départ d'une souche utilisée dans l'industrie de panification.

Cette souche TGF1 étant polyploïde et prototrophe, on doit effectuer une cotransformation avec le pTG1867 et un plasmide conférant un phénotype dominant, le plasmide pTG1825.

La souche TGF1 est transformée avec l'ADN des plasmides pTG1825 et pTG1867 préalablement digéré par EcoRI. Parmi les 500 clones résistant au G418 (300 µg/ml) obtenus, l'un exprime l'amyloglucosidase de façon stable durant au moins 50 générations ; ce clone est appelé TGF1-AMG1.

L'hybridation des ADN génomiques digérés par EcoRI des souches TGF1-AMG1 et TGF1 par un oligonucléotide TG529 spécifique du gène MFalphaI de Saccharomyces cerevisiae par la technique de Southern révèle que le bloc d'expression de l'amyloglucosidase est intégré dans le génome au locus MFalphaI (figure 15) de TGF1-AMG1.

La quantité d'amyloglucosidase sécrétée par la souche TGF1-AMG1 a été dosée. Après trois jours d'incubation en milieu complet YPG à 30°C sous agitation à 250 RPM, la culture de TGF1-AMG1 produit 100 U d'alpha-amylglucosidase active par litre de surnageant d'une culture à une densité cellulaire de 5 x 10$^7$ cellules/ml.

EXEMPLE 10

INTEGRATION DANS LA SOUCHE POLYPLOIDE SACCHAROMYCES UVARUM 2285 TG2 AMGC DU BLOC D'EXPRESSION DE L'ALPHAAMYLASE DE B. LICHENIFORMIS

On a transformé la souche 2285 TG2 AMGC avec 1 µg d'ADN de pTG1825 et 10µg de M13TG1817 préalablement digéré par BamHI par la technique dérivée de Webster et Dickson (18).

Le vecteur M13TG1817 libère par digestion avec BamHI un fragment d'ADN contenant le gène His3 de Saccharomyces uvarum dans lequel on a inséré la cassette d'expression de l'alpha-amylase. La construction du vecteur M13TG1817 a été décrite dans le brevet français no 86 06703.

Sur 400 clones résistant au G418 (300 µg/ml), 4 clones produisent de l'alpha-amylase. Ces clones expriment l'alpha-amylase de façon stable durant 50 générations. L'hybridation des ADN génomiques digérés par BamHI des souches 2285 TG2 AMGC-AMY1 et 2285 TG2 AMGC par un oligonucléotide TG227 spécifique du gène His3 de S. uvarum par la technique de Southern révèle que le bloc d'expression de l'alpha-amylase est intégré dans le génome au locus His3 (figure 16) de la souche 2285 TG2 AMGC-AMY1.

Les souches suivantes ont été déposées à la Collection Nationale de Cultures de Microorganismes de l'Institut Pasteur, 28 rue du Docteur-Roux - 75724 PARIS Cédex 15 :
. Saccharomyces uvarum 2285 TG2 AMGC le 6 juin 1986 sous le no 1-568,
. Saccharomyces cerevisiae TGF1 AMG1 le 27 janvier 1987 sous le no 1-650,
. Saccharomyces uvarum 2285 TG2 AMGC-AMY1 le 27 janvier 1987 sous le no 1-649.

REFERENCES

1. Brake, A.J., Merryweather, J.P., Coit, D.C., Heberlein, U.A., Mariars, F.R., Mullenbach, G.T., Urdea, M.S., Valenzuela, P. and Barr, P.J. (1984) Proc. Natl. Acad. Sci. USA 81, 4642-4646.

2. Bussey, H., Saville, D., Greene, D. et al. (1983) Mol. Cell. Biol. 3, 1362-1970.

3. Grunstein M. and Hogness, D.S. (1975) Colony hybridization : A method for the isolation of cloned DNAs that contain a specific gene. Proc. Natl. Acad. Sci. USA 72, 10, 4961-3965.

4. a) Boel, E., Hansen, M.T., Hjort, J., Hoegh, I. and Fiil, N.P. (1984) Embo Journal vol. 3, 7, 1581-1585.
b) Boel, E., Hjort, I., Svensson, B., Norris, F., Norris, K.E. and Fiil, N.P. (1984) Embo Journal vol. 3, 5, 1097-1102.

5. Langford, C.J. and Gallwitz, D. (1983) Cell 33, 519-527.

6. Chang, A.C.Y., Nunberg, J.H., Kaufman, R.M.J., Erlich, M.A., Schimke, R.T. and Cohen, S.N. (1978) Nature 275, 617-624.

7. Le Bouc, Y., Dreyer, D., Jaeger, F., Binoux, M. and Sondermeyer, P. (1986) FEBS vol.196, 1, 108-112.

8. Zollet, M. J. and Smith, M.H. (1983) Meth. in Enzym. 100, 469.

9. Beggs, J. (1981) Genetic Engineering 2, 175-203.

10. Broach, J.R. (1981) in the Molecular Biology of the Yeast Saccharomyces. Life cycle and Inheritance CHS press, New York.

11. Bach, M.L., Lacroute, F. and Botstein, D. (1979) Proc. Natl. Acad. Sci. (USA) 76, 386-390.

12. Sutcliffe, J.G. (1978) Proc. Natl. Acad. Sci. USA 75, 3737-3741.

13. Hitzeman, R.A., Hagie, E.F., Mayfflick, J.S. et al. (1982) Nucleic Acids Res. 10, 7791-7808.

14. Ito, H., Fukuda, Y., Murata, K. and Kimura, A. (1983) J. Bacteriol. 153, 163-168.

15. Chevallier, M.R., Bloch, J.C. and Lacroute, F. (1980) Gene 11, 11-19.

16. Kurjan, J. (1985) Molecular and Cellular Biology 5, 787-796.

17. Brake, A.S., Julius, D.S. and Thorner, J. (1983) Molecular and cellular Biology 3, 1440-1450.

18. Webster, T.D. and Dickson, R.C. (1983) 26, 243-252.

**Revendications**

1) Bloc d'expression d'une amyloglucosidase dans une levure, charactérisé en ce qu'il comporte :
. le gène de l'amyloglucosidase dépourvu de tout ou partie de ses introns ;
. la séquence d'ADN contenant les signaux assurant la transcription du gène de l'amyloglucosidase par la levure ;
. une séquence codante contenant le signal nécessaire à l'export de l'amyloglucosidase mature dans le milieu extracellulaire.

2) Bloc d'expression selon la revendication 1, caractérisé en ce qu'il comporte le gène de l'amyloglucosidase dépourvu de ses introns.

3) Bloc d'expression selon l'une des revendications 1 et 2, caractérisé en ce que la séquence codante contenant le signal nécessaire à l'export de l'amyloglucosidase mature contient un signal de sécrétion choisi parmi la phéromone alpha ou bien le système de sécrétion de la protéine "killer" ou bien une séquence de sécrétion synthétique.

4) Bloc d'expression selon l'une des revendications 1 à 3, caractérisé en ce qu'il comporte, en outre, après le gène de l'amyloglucosidase, une séquence de terminaison de levure.

5) Bloc d'expression selon l'une des revendications 1 à 4, caractérisé en ce que la séquence d'ADN assurant la transcription du gène comporte le promoteur du gène PGK.

6) Bloc d'expression selon l'une des revendications 1 à 5, caractérisé en ce qu'il est intégré dans un plasmide comportant une origine de réplication dans les levures.

7) Bloc d'expression selon la revendication 6, caractérisé en ce que l'origine de réplication dans les levures est l'origine de réplication du plasmide 2μ.

8) Bloc d'expression selon l'une des revendications 6 et 7, caractérisé en ce que le plasmide comporte, en outre, un gène de sélection.

(9) Bloc d'expression selon la revendication 8, caractérisé en ce que le gène de sélection est un gène complémentant une auxotrophie.

10) Bloc d'expression selon la revendication 8, caractérisé en ce que le gène de sélection est un gène conférant un phénotype dominant.

11) Bloc d'expression selon la revendication 10, caractérisé en ce que le phénotype dominant est la résistance au G418.

12) Bloc d'expression selon l'une des revendications 1 à 6, caractérisé en ce qu'il est intégré dans le génome de la souche de levure.

13) Bloc d'expression selon la revendication 12, caractérisé en ce que l'intégration est effectuée dans le gène MFalpha,.

14) Souche de levure transformée comportant un bloc d'expression selon l'une des revendications 1 à 13.

15) Souche selon la revendication 14, caractérisée en ce qu'il s'agit d'une souche de Saccharomyces.

16) Souche selon la revendication 15, caractérisée en ce qu'il s'agit d'une souche choisie parmi : S. cerevisiae et S. uvarum.

17) Souche transformée selon l'une des revendications 14 à 16, caractérisée en ce qu'il s'agit d'une souche de Saccharomyces polyploïde et prototrophe.

18) Souche selon la revendication 17, caractérisée en ce qu'il s'agit d'une souche de Saccharomyces utilisée dans l'industrie de panification.

19) Souche selon la revendication 18, caractérisée en ce qu'il s'agit de la souche de Saccharomyces cerevisiae TGF1.

20) Souche selon l'une des revendications 17 à 19, caractérisée en ce que le bloc d'expression de l'amyloglucosidase dérive du plasmide pTG1867.

21) Souche de Saccharomyces selon l'une des revendications 14 à 16, caractérisée en ce qu'elle est transformée simultanément par un bloc d'expression selon l'une des revendications 1 à 13 et par un bloc d'expression de l'alpha-amylase et en ce qu'elle sécrète simultanément l'alpha-amylase et l'amyloglucosidase.

22) Souche selon la revendication 21, caractérisée en ce qu'il s'agit d'une souche brassicole du genre Saccharomyces uvarum.

23) Souche selon la revendication 22, caractérisée en ce qu'il s'agit de la souche S. uvarum 2285 TG2 AMGC.

24) Souche selon l'une des revendications 21 à 23, caractérisée en ce que le bloc d'expression de l'alpha-amylase dérive du vecteur M13TG1817.

25) Procédé de production d'alpha-amyloglucosidase, caractérisé en ce qu'on fait fermenter sur un milieu de culture approprié une souche selon l'une des revendications 14 à 24 et en ce qu'on récupère l'enzyme dans le milieu extracellulaire.

26) Procédé de fermentation panaire, caractérisé en ce qu'au moins l'une des souches utilisées dans la fermentation est une souche selon l'une des revendications 17 à 20.

27) Procédé de fermentation caractérisé en ce qu'au moins l'une des souches utilisées dans la fermentation est une souche selon l'une des revendications 21 à 24.

28) Procédé de dégradation de l'amidon ou des dextrines, caractérisé en ce qu'on fait fermenter un milieu de fermentation contenant de l'amidon ou des dextrines.

0260160

Gène glucoamylase de A. niger  (a)

PTG 1831
4712 bp
(b)

PTG 1830
6110 bp
(c)

## FIG_1

FIG. 2

TTCGTCGCCTAATGTCTCGTCCGTTCACAAACTG

AAGAGCTTGAAGTGGCGAGATGTCTCTGCAGGAATTC
EcoRI

AAGCTAGATGCTAAGCGATATTGCAT GGCAATAT GTGTT

GATGCATGTGCTTCTTCCTTCAGCTTCCCCTCGTGCGAGTGA

GGTTTGGC TATAAAT TGAAGTGGTTGGTCGGGGTTC

CGTGAGGGGCTGAAGTGCTTCCTCCCTTTTAGGCGCAAC

TGAGAGCCTGAGCTTCATCCCCAGCATCATTACACCTCAGCA

| ATG | TCG | TTC | CGA | TCT | CTA | CTC | GCC | CTG | AGC |
|-----|-----|-----|-----|-----|-----|-----|-----|-----|-----|
| Met | Ser | Phe | Arg | Ser | Leu | Leu | Ala | Leu | Ser |

| GGC | CTC | GTC | TGC | ACA | GGG | TTG | GCA | AAT | GTG |
|-----|-----|-----|-----|-----|-----|-----|-----|-----|-----|
| Gly | Leu | Val | Cys | Thr | Gly | Leu | Ala | Asn | Val |

| ATT | TCC | AAG | CGC | GCG | ACC | TTG | GAT | TCA | TGG |
|-----|-----|-----|-----|-----|-----|-----|-----|-----|-----|
| Ile | Ser | Lys | Arg | Ala | Thr | Leu | Asp | Ser | Trp |

| TTG | AGC | AAC | GAA | GCG | ACC | GTG | GCT | CGT | ACT |
|-----|-----|-----|-----|-----|-----|-----|-----|-----|-----|
| Leu | Ser | Asn | Glu | Ala | Thr | Val | Ala | Arg | Thr |

| GCC | ATC | CTG | AAT | AAC | ATC | GGG | GCG | GAC | GGT |
|-----|-----|-----|-----|-----|-----|-----|-----|-----|-----|
| Ala | Ile | Leu | Asn | Asn | Ile | Gly | Ala | Asp | Gly |

FIG. 2

GCT TGG GTG TCG GGC GCG GAC TCT GGC ATT[60]
Ala Trp Val Ser Gly Ala Asp Ser Gly Ile

GTC GTT GCT AGT CCC AGC ACG GAT AAC CCG[70]
Val Val Ala Ser Pro Ser Thr Asp Asn Pro

GAC T GTATGTTTCGAGCTCAGATTTAGTATGAGTGTGTC
Asp                                                IVS

ATTGATTGATTGAT<u>GCTGACTGG</u>CGTGTCGTTTGTTGTAG
A

AC TTC TAC ACC TGG ACT CGC GAC TCT[80] GGT
Tyr Phe Tyr Thr Trp Thr Arg Asp Ser Gly

CTC GTC CTC AAG ACC CTC GTC GAT CTC[90] TTC
Leu Val Leu Lys Thr Leu Val Asp Leu Phe

CGA AAT GGA GAT ACC AGT CTC CTC TCC[100] ACC
Arg Asn Gly Asp Thr Ser Leu Leu Ser Thr

ATT GAG AAC TAC ATC TCC GCC CAG GCA[110]
Ile Glu Asn Tyr Ile Ser Ala Gln Ala

ATT GTC CAG GGT ATC AGT AAC CCC TCT GGT[120]
Ile Val Gln Gly Ile Ser Asn Pro Ser Gly

GAT CTG TCC AGC GGC GCT GGT CTC GGT GAA[130]
Asp Leu Ser Ser Gly Ala Gly Leu Gly Glu

FIG_2

```
                                                              140
CCC  AAG TTC AAT GTC GAT GAG ACT GCC TAC
Pro  Lys Phe Asn Val Asp Glu Thr Ala Tyr

                                                              150
ACT GGT TCT TGG GGA CGG CCG CAG CGA GAT
Thr Gly Ser Trp Gly Arg Pro Gln Arg Asp

                                                              160
GGT CCG GCT CTG AGA GCA ACT GCT ATG ATC
Gly Pro Ala Leu Arg Ala Thr Ala Met Ile

GGC  TTC GGG CAG TGG CTG CTT GTATGTTCTCCAC
Gly  Phe Gly Gln Trp Leu Leu
```

CCCCTTGCGTCTGATCTGTGACATATGTAGCTGACTGGTCAG
    IVS B

```
     170
GAC AAT GGC TAC ACC AGC ACC GCA ACG GAC
Asp Asn Gly Tyr Thr Ser Thr Ala Thr Asp
                        TG433

ATT GTT TGG CCC CTC GTT AGG AAC GAC CTG
Ile Val Trp Pro Leu Val Arg Asn Asp Leu

     190
TCG TAT GTG GCT CAA TAC TGG AAC CAG ACA
Ser Tyr Val Ala Gln Tyr Trp Asn Gln Thr

GGA TAT G  GTGTGTTTGTTTTATTTTAAATTTCCAAAGA
Gly Tyr                              IVS C
```

                                                          200
TGCGCCAGCAGAGCTAACCCGCGATCGCAG  AT CTC
                                   Asp Leu

0260160

FIG_2

```
                                              210
TGG GAA GAA GTC AAT GGC TCG TCT TTC TTT
Trp Glu Glu Val Asn Gly Ser Ser Phe Phe

                                              220
ACG ATT GCT GTG CAA CAC CGC GCC CTT GTC
Thr Ile Ala Val Gln His Arg Ala Leu Val

                                              230
GAA GGT AGT GCC TTC GCG ACG GCC GTC GGC
Glu Gly Ser Ala Phe Ala Thr Ala Val Gly

                                              240
TCG TCC TGC TCC TGG TGT GAT TCT CAG GCA
Ser Ser Cys Ser Trp Cys Asp Ser Gln Ala
```

TG387

```
                                              250
CCC GAA ATT CTC TGC TAC CTG CAG TCC TTC
Pro Glu Ile Leu Cys Tyr Leu Gln Ser Phe

                                              260
TGG ACC GGC AGC TTC ATT CTG GCC AAC TTC
Trp Thr Gly Ser Phe Ile Leu Ala Asn Phe

                                              270
GAT AGC AGC CGT TCC GGC AAG GAC GCA AAC
Asp Ser Ser Arg Ser Gly Lys Asp Ala Asn

                                              280
ACC CTC CTG GGA AGC ATC CAC ACC TTT GAT
Thr Leu Leu Gly Ser Ile His Thr Phe Asp

                                              290
CCT GAG GCC GCA TGC GAC GAC TCC ACC TTC
Pro Glu Ala Ala Cys Asp Asp Ser Thr Phe

                                              300
CAG CCC TGC TCC CCG CGC GCG CTC GCC AAC
Gln Pro Cys Ser Pro Arg Ala Leu Ala Asn

                                              310
CAC AAG GAG GTT GTA GAC TCT TTC CGC TCA
His Lys Glu Val Val Asp Ser Phe Arg Ser
```

FIG. 2

```
ATC  TAT  ACC  CTC  AAC  GAT  GGT  CTC  AGT  GAC
                                        320
Ile  Tyr  Thr  Leu  Asn  Asn  Gly  Leu  Ser  Asp

AGC  GAG  GCT  GTT  GCG  GTG  GGT  CGG  TAC  CCT
                                        330
Ser  Glu  Ala  Val  Ala  Val  Gly  Arg  Tyr  Pro

GAG  GAC  ACG  TAC  TAC  AAC  GGC  AAC  CCG  TGG
                                        340
Glu  Asp  Thr  Tyr  Tyr  Asn  Gly  Asn  Pro  Trp ──TG283

TTC  CTG  TGC  ACC  TTG  GCT  GCC  GCA  GAG  CAG
                                        350
Phe  Leu  Cys  Thr  Leu  Ala  Ala  Ala  Glu  Gln

TTG  TAC  GAT  GCT  CTA  TAC  CAG  TGG  GAC  AAG
                                        360
Leu  Tyr  Asp  Ala  Leu  Tyr  Gln  Trp  Asp  Lys

CAG  GGG  TCG  TTG  GAG  GTC  ACA  GAT  GTG  TCG
                                        370
Gln  Gly  Ser  Leu  Glu  Val  Thr  Asp  Val  Ser

CTG  GAC  TTC  TTC  AAG  GCA  CTG  TAC  AGC  GAT
                                        380
Leu  Asp  Phe  Phe  Lys  Ala  Leu  Tyr  Ser  Asp

GCT  GCT  ACT  GGC  ACC  TAC  TCT  TCG  TCC  AGT
                                        390
Ala  Ala  Thr  Gly  Thr  Tyr  Ser  Ser  Ser  Ser

TCG  ACT  TAT  AGT  AGC  ATT  GTA  GAT  GCC  GTG
                                        400
Ser  Thr  Tyr  Ser  Ser  Ile  Val  Asp  Ala  Val

AAG  ACT  TTC  GCC  GAT  GGC  TTC  GTC  TCT  ATT
                                        410
Lys  Thr  Phe  Ala  Asp  Gly  Phe  Val  Ser  Ile

GTG  GTAAGTCTACGCTAGACAAGCGCTCATGTTGACAG
Val                                IVS  D
```

FIG_2

```
AGGGTGCGTACTAACAGAAGTAG  GAA ACT CAC GCC
                         Glu Thr His Ala

         420
GCA AGC AAC GGC TCC ATG TCC GAG CAA TAC
Ala Ser Asn Gly Ser Met Ser Glu Gln Tyr
   —TG496
         430
GAC AAG TCT GAT GGC GAG CAG CTT TCC GCT
Asp Lys Ser Asp Gly Glu Gln Leu Ser Ala

         440
CGC GAC CTG ACC TGG TCT TAT GCT GCT CTG
Arg Asp Leu Thr Trp Ser Tyr Ala Ala Leu

         450
CTG ACC GCC AAC AAC CGT CGT AAC TCC GTC
Leu Thr Ala Asn Asn Arg Arg Asn Ser Val

         460
GTG CCT GCT TCT TGG GGC GAG ACC TCT GCC
Val Pro Ala Ser Trp Gly Glu Thr Ser Ala

         470
AGC AGC GTG CCC GGC ACC TGT GCG GCC ACA
Ser Ser Val Pro Gly Thr Cys Ala Ala Thr

         480
TCT GCC ATT GGT ACC TAC AGC AGT GTG ACT
Ser Ala Ile Gly Thr Tyr Ser Ser Val Thr

         490
GTC ACC TCG TGG CCG AGT ATC GTG GCT ACT
Val Thr Ser Trp Pro Ser Ile Val Ala Thr

         500
GGC GGC ACC ACT ACG ACG GCT ACC CCC ACT
Gly Gly Thr Thr Thr Thr Ala Thr Pro Thr
```

FIG_2

```
                    510
GGA TCC GGC AGC GTG ACC TCG ACC AGC AAG
Gly Ser Gly Ser Val Thr Ser Thr Ser Lys

                    520
ACC ACC GCG ACT GCT AGC AAG ACC AGC ACC
Thr Thr Ala Thr Ala Ser Lys Thr Ser Thr

                    530
AGT ACG TCA TCA ACC TCC TGT ACC ACT CCC
Ser Thr Ser Ser Thr Ser Cys Thr Thr Pro

                    540
ACC GCC GTG GCT GTG ACT TTC GAT CTG ACA
Thr Ala Val Ala Val Thr Phe Asp Leu Thr

                    550
GCT ACC ACC ACC TAC GGC GAG AAC ATC TAC
Ala Thr Thr Thr Tyr Gly Glu Asn Ile Tyr

                    560
CTG GTC GGA TCG ATC TCT CAG CTG GGT GAC
Leu Val Gly Ser Ile Ser Gln Leu Gly Asp

                    570
TGG GAA ACC AGC GAC GGC ATA GCT CTG AGT
Trp Glu Thr Ser Asp Gly Ile Ala Leu Ser
```

**TG284**

```
                    580
GCT GAC AAG TAC ACT TCC AGC GAC CCG CTC
Ala Asp Lys Tyr Thr Ser Ser Asp Pro Leu

                    590
TGG TAT GTC ACT GTG ACT CTG CCG GCT GGT
Trp Tyr Val Thr Val Thr Leu Pro Ala Gly

                    600
GAG TCG TTT GAG TAC AAG TTT ATC CGC
Glu Ser Phe Glu Tyr Lys Phe Ile Arg
```

FIG_2

|     |     |     |     | 610 |     |     |     |     |     |
| ATT | GAG | AGC | GAT | GAC | TCC | GTG | GAG | TGG | GAG |
| Ile | Glu | Ser | Asp | Asp | Ser | Val | Glu | Trp | Glu |

|     |     |     |     | 620 |     |     |     |     |     |
| AGT | GAT | CCC | AAC | CGA | GAA | TAC | ACC | GTT | CCT |
| Ser | Asp | Pro | Asn | Arg | Glu | Tyr | Thr | Val | Pro |

|     |     |     |     | 630 |     |     |     |     |     |
| CAG | GCG | TGC | GGA | ACG | TCG | ACC | GCG | ACG | GTG |
| Gln | Ala | Cys | Gly | Thr | Ser | Thr | Ala | Thr | Val |

|     |     |     |     | 640 |     |          |
| ACT | GAC | ACC | TGG | CGG | TGA | CAATCAATCCATTTCGCT |
| Thr | Asp | Thr | Trp | Arg | Stop |            |

ATAGTTAAAGGATGGGGATGAGGGCAATTGGTTATATGATCATG
TG282

TATGTAGTGGGTGTGCATAATAGTAGTGAAATGGAAGCCA

AGTCATGTGATTGTAATCGACCGACGGAATTGAGGATATC
EcoRV

CGGAAATACAGACACCGGG

M13tg 1839

| | |
|---|---|
| ▨▨▨ | M13mp8 |
| ☐ | CDNA<br>Sequence codant pour<br>les aminoacides 62-219 |

## FIG_3

PTG 1830 digéré par Pst I et Sal I

libération d'un fragment de 1,2 KB

PTG 1831 digéré par Eco R I et Sal I

libération d'un fragment de 1 KB

Ligation avec le M13mp8 digéré par PstI et EcorI

Transformation E. coli

M13Tg 1843 :

BssH2

Ecor $\overline{\text{V}}$

intron D

Pst I

EcorI

SalI

///// Séquence de M13mp 8

☐ Séquence de la partie 3' du gène de l'amyloglucosidase

FIG_4

M13Tg 1835 :

BamH I    Pst I    SacI    EcoRI

D    C    B    A

Séquence intron A : Hybridation du simple brin de M13Tg 1835 avec l'oligo Tg 403 :

5' - ACGGATAACCCGGACTACTTCTACACCT- 3'
3' ......GGGTCGTGCCTATTGGGCCTGATGAAGATGTGGACCTGAG... ...5'

SacI

Elongation du brin complémentaire par la
        polymérase klenow
Ligation
Digestion sac I
Transformation E. coli

M13Tg 1837 :

BamHI    Pst I    AvaII    BssHII    EcoRI

D    C    B

Séquence de M13Tg 127

Séquence de la partie 5' du gène de
l'amyloglucosidase

FIG_5

M13Tg 1837 digéré par BssHII et AvaII

Isolement d'un fragment de 380 bp.

M13Tg 1839 digéré par Pst I et AvaII

Isolement d'un fragment de 280 bp

BssHII

```
          Ala  Ser  Leu  Asp  Lys
+ 2 oligonucléotides synthétiques : Tg 453:  A  GCT  TCT  TTG  GAT  AAG
                                     Tg 454:     AGA  AAC  CTA  TTC  GCG
```

HindIII

Ligation dans le M13mp8 digéré par Hind III et Pst I

Transformation E. coli

Pst I

BssHII

HindIII

AvaII

M13Tg 1846 :

280bp    380bp

oligonucléotides synthétiques

## FIG.6

0260160

0260160

M13Tg 1846 digéré par Hind III et Pst I

Isolement d'un fragment de 670 bp
codant pour les acides aminés
21 a 246

M13Tg 1843 digéré par Pst I et EcoRV

Isolement de la partie 3' du gène de
l'amyloglucosidase sous forme d'un fragment
de 1376 bp codant pour les acides aminés
246 á 640

Ligation dans le M13Tg 131 digéré par Hind III et Hind II

Transformation de E. coli

M13Tg 1848 :

EcoRV°/ Hind II°

Pst I

BglII

intron D
1376 bp

Pst I

670 bp

HindIII

## FIG_7

0260160

ptG848

FIG_8a

PTG 1858

FIG_10

0260160

PTG 848 digéré par BglII
= le vecteur

M13 1848 digéré par BglII
et HindIII

Isolement du fragment
de 2046 bp

+ M13Tg 889

BamHI          HindIII          SalI

( 26/bp )

digéré par HindIII et BamHI

Isolement du fragment de 267 bp

Ligation

Transformation E. coli

HindIII

HindIII                    Promoteur PGK

BglII°/BamHI°
pré-pro
phéromone
HindIII

PTG 1850

gène de
l'amylo-         PstI
glucosidase      SphI

intron D

BamHI

Leu 2
EcoRI

BglII
Terminateur
PGK

EcoRI                 Amp      ori

PstI

FIG_8b

0260160

Séquence intron D : hybridation du simple brin de M13tg 1847 avec l'oligo TG 496

5' TTCGTCTCTATTGTGGAAACTCACGCCGCA 3'
CCG AAGCAGAGATAACACCTTTGAGTGCGGCGT TCGTTG

intron D

Elongation du brin complémentaire par la polymérase Klenow

Ligation

Transformation E.coli

M13 tg 1857

Séquence de M13 tg 127

Séquence codant pour le précurseur prepro-amyloglucosidase

## FIG. 9

FIG_11

0260160

digestion par <u>Hind II</u>

fragment Sma I - Bgl II du
pfG 1858
fragment Bgl II - Hind III du pfG 830
traitement Klenow sur
Site Hind III

Ligation

pfG 1867

## FIG_12

| | Séquence pfG 1865 |
| | Séquences fragment MFα1 de S. cérévisi |
| | Séquences 5' et 3' du gène PGK |
| | Séquences codant pour l'amyloglucosidase |
| | Séquence codant pour partie PrePro du gene MFα1 |

0260160

MFα1 gene disrupté par
le bloc d'expression

MFα1 gene (1,7kb)

FIG_13

0260160

TG 170

Correspondant à la séquence
entre les sites Hind II

TG 529

Correspondant à la séquence
3' du gene MFα1

FIG_14

0260160

# FIG_15

A  B  C

← gène MFα1 contenant le bloc d'expression de l'AMG

← gène MFα1

A  TGF1

B  TGF1-AMG1 Eco RI

C  pTG 1867 Eco RI

0260160

## FIG_16

A    B        C

← gène His3 contenant
le bloc d'expression
de l'α – amylase

← gène His3

A   2285 TG2 AMGC
B   2285 TG2 AMGC–AMY1
C   M13TG1817

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl 4) |
|---|---|---|---|
| X | EP-A-0 126 206 (CETUS CORP.)<br><br>* Page 2 - page 3, ligne 14; page 4, ligne 26 - page 5, ligne 3; page 5, ligne 24 - page 6, ligne 3; page 11, ligne 5 - page 12, ligne 4; page 12, ligne 3 - page 14, ligne 28; pages 33-36; page 43, ligne 20 - page 47, ligne 6; revendications 4,7,8,9,19-25; page 50, lignes 10-16; page 10, ligne 28 - page 11, ligne 4 * | 1,2,4-9,12-16,25,28 | C 12 N 15/00<br>C 12 N 1/18<br>C 12 N 9/34<br>C 12 P 19/20<br>A 21 D 8/04 |
| Y | | 3,10,17-24,26,27 | |
| X | --- <br>EP-A-0 163 491 (BIOTECHNICA)<br><br>* Page 1, ligne 1 - page 3, ligne 26; page 4, ligne 6 - page 5, ligne 12; page 8, lignes 10-13; page 9, ligne 26 - page 10, ligne 18; page 10, ligne 20 - page 12, ligne 10; revendications 9,10,12-20; figures 1-4 *<br><br>---      -/- | 1,2,6-12,14-18,25,26,28 | **DOMAINES TECHNIQUES RECHERCHES (Int. Cl.4)**<br><br>C 12 N<br>C 12 D<br>A 21 D |

Le présent rapport de recherche a été établi pour toutes les revendications

| Lieu de la recherche | Date d'achèvement de la recherche | Examinateur |
|---|---|---|
| LA HAYE | 12-10-1987 | MADDOX A.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

## DOCUMENTS CONSIDERES COMME PERTINENTS

Page 2

| Catégorie | Citation du document avec indication, en cas de besoin, des parties pertinentes | Revendication concernée | CLASSEMENT DE LA DEMANDE (Int Cl 4) |
|---|---|---|---|
| Y | | 3-5,13 ,19-24 ,27 | |
| | --- | | |
| X | AGRICULTURAL AND BIOLOGICAL CHEMISTRY, vol. 50, no. 4, avril 1986, pages 957-964, Tokyo, JP; T. ASHIKARI et al.: "Rhizopus raw-starch-degrading glucoamylase: its cloning and expression in yeast" * En entier * | 1,2,4, 6-9,14 -16,25 ,28 | |
| A | Idem | 3,5,10 -13,17 -24,26 ,27 | |
| | --- | | DOMAINES TECHNIQUES RECHERCHES (Int. Cl.4) |
| X | PROC. CONGR. EUR. BREW. CONV. 1985, pages 219-226; P.G. MEADEN et al.: "A plasmid vector system for the genetic manipulation of brewing strains" * Page 224, "Construction of a dextrin-fermenting brewing strain by transformation" - page 225 * | 1,2,4, 6-10, 14-17, 25,28 | |
| Y | Idem | 3,5,11 -13,18 -24,26 ,27 | |

-/-

Le présent rapport de recherche a été établi pour toutes les revendications

| Lieu de la recherche | Date d'achèvement de la recherche | Examinateur |
|---|---|---|
| LA HAYE | 12-10-1987 | MADDOX A.D. |

| | **DOCUMENTS CONSIDERES COMME PERTINENTS** | | Page   3 |
|---|---|---|---|

| Catégorie | Citation du document avec indication, en cas de besoin, des parties pertinentes | Revendication concernée | CLASSEMENT DE LA DEMANDE (Int. Cl.4) |
|---|---|---|---|
| Y | BIOTECHNOLOGY, vol. 4, no. 4, avril 1986, pages 311-315, New York, US; S.A. FILHO et al.: "Stable yeast transformants that secrete functional alpha-amylase encoded by cloned mouse pancreatic cDNA" <br> *   En entier, notamment page 314, colonne de gauche, dernier paragraphe * <br> --- | 3,21-25,27, 28 | |
| Y | EP-A-0 123 294  (AMGEN) <br> *   Page  10,  ligne 15 - page 11, ligne 19; page 18, lignes  14-22; revendications 1-4 * <br> --- | 3,5 | |
| Y | BIOTECHNOLOGY AND GENETIC ENGINEERING REVIEWS, vol. 3, septembre 1985, pages 377-416, Intercept Ltd; S.M. KINGSMAN et al.: "Heterologous gene expression in Saccharomyces cerevisiae" <br> *   Page  378, paragraphe 2 - page 384; page  388,  paragraphe  5  - page  392; page 398, paragraphe 2 - page 403; pages  406-407,  "Future prospects" * <br> ---                 -/- | 1-12 | DOMAINES TECHNIQUES RECHERCHES (Int. Cl.4) |

Le présent rapport de recherche a été établi pour toutes les revendications

| Lieu de la recherche | Date d'achèvement de la recherche | Examinateur |
|---|---|---|
| LA HAYE | 12-10-1987 | MADDOX A.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

&  : membre de la même famille, document correspondant

OEB Form 1503 03 82

| Catégorie | Citation du document avec indication, en cas de besoin, des parties pertinentes | Revendication concernée | CLASSEMENT DE LA DEMANDE (Int. Cl 4) |
|---|---|---|---|
| | **DOCUMENTS CONSIDERES COMME PERTINENTS** | | Page 4 |
| Y | APPLIED AND ENVIRONMENTAL MICROBIOLOGY, vol. 47, no. 5, mai 1984, pages 1164-1166, American Society for Microbiology; C.J. PANCHAL et al.: "Susceptibility of Saccharomyces spp. and Schwanniomyces spp. to the aminoglycoside antibiotic G418" * Abrégé; page 1164, colonne de gauche, paragraphe 1 * --- | 10,11, 21-24, 27 | |
| Y | FOUNDATION FOR BIOTECHNICAL AND INDUSTRIAL FERMENTATION RESEARCH VI, 1983, pages 209-228; A.M. SILLS et al.: "Genetic manipulation of amylolytic enzyme production by yeasts" * Page 210, paragraphes 2,3; page 220, lignes 5-8; page 223 * --- | 21-25, 27 | |
| Y | GENE, vol. 36, no. 1/2, 1985, pages 87-95, Elsevier Science Publishers, Amsterdam, NL; H. RUDOLPH et al.: "One-step gene replacement in yeast by cotransformation" * En entier * --- | 12,13 | DOMAINES TECHNIQUES RECHERCHES (Int. Cl.4) |
| Y | MOLECULAR AND CELLULAR BIOLOGY, vol. 5, no. 4, avril 1985, pages 787-796, American Society for Micriobiology; J. KURJAN: "Alpha-factor structural gene mutations in Saccharomyces cerevisiae: effects on alpha-factor production and mating" * En entier * --- | 12,13 | |

Le présent Rapport de recherche a été établi pour toutes les revendications

| Lieu de la recherche | Date d'achèvement de la recherche | Examinateur |
|---|---|---|
| LA HAYE | 12-10-1987 | MADDOX A.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

| Catégorie | Citation du document avec indication, en cas de besoin, des parties pertinentes | Revendication concernée | CLASSEMENT DE LA DEMANDE (Int. Cl 4) |
|---|---|---|---|
| | **DOCUMENTS CONSIDERES COMME PERTINENTS** | | Page   5 |
| Y | FOOD TECHNOLOGY, vol. 18, no. 10, octobre 1964, pages 95-148; Y. POMERANZ et al.: "Use of amyloglucosidase in breadmaking" * En entier * --- | 18,26 | |
| Y | CHEMICAL ABSTRACTS, vol. 88, no. 15, avril 1978, page 412, résumé no. 103355r, Columbus, Ohio, US; & JP-A-77 130 977 (ORIENTAL YEAST CO. LTD) 02-11-1977 * Résumé * --- | 18,26 | |
| A | WO-A-8 404 539  (TRANSGENE) * Revendications 5-13,23,25,26 * --- | 1-28 | |
| A | THE EMBO JOURNAL, vol. 3, no. 5, 1984, pages 1097-1102; E. BOEL et al.: "Glucoamylases G1 and G2 from Aspergillus niger are synthesized from two different but closely related mRNAs" * Résumé * --- | | DOMAINES TECHNIQUES RECHERCHES (Int. Cl.4) |
| A | AGRICULTURAL AND BIOLOGICAL CHEMISTRY, vol. 50, no. 5, mai 1986, pages 1177-1182, Tokyo, JP; K. SAKAI et al.: "Transformation of the yeast, Saccharomyces carlsbergensis, using an antibiotic resistance marker" * En entier * ---                  -/- | 10,11, 15-17 | |

Le présent rapport de recherche a été établi pour toutes les revendications

| Lieu de la recherche | Date d'achèvement de la recherche | Examinateur |
|---|---|---|
| LA HAYE | 12-10-1987 | MADDOX A.D. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

Office européen
des brevets

**RAPPORT DE RECHERCHE EUROPEENNE**

Numero de la demande

EP 87 40 1300

| Catégorie | Citation du document avec indication, en cas de besoin, des parties pertinentes | Revendication concernée | CLASSEMENT DE LA DEMANDE (Int Cl 4) |
|---|---|---|---|
| | **DOCUMENTS CONSIDERES COMME PERTINENTS** | | Page 6 |
| E | WO-A-8 603 778 (BREWING RESEARCH FOUNDATION) <br><br> * Revendications 1-10; page 26, paragraphes 1,2 * <br> --- | 1,2,4-9,14-17,25,27,28 | |
| E | EP-A-0 185 327 (SUNTORY) <br><br> * Page 1, ligne 31 - page 2, ligne 2; page 2, ligne 38 - page 3, ligne 28; page 6, ligne 8 - page 7, ligne 4; page 10, ligne 3 - page 13, ligne 12; page 13, ligne 32 - page 16, ligne 25; page 19, ligne 36 - page 20, ligne 11; revendications; figure 6A * <br> --- | 1,2,4-9,14-16,25,27,28 | |
| E | WO-A-8 607 091 (BIOTECHNICA) <br><br> * En entier * <br> --- <br><br> ----- | 1,2,4,8-12,14-18,25,27,28 | **DOMAINES TECHNIQUES RECHERCHES (Int. Cl.4)** |

Le présent rapport de recherche a été établi pour toutes les revendications

| Lieu de la recherche | Date d'achèvement de la recherche | Examinateur |
|---|---|---|
| LA HAYE | 12-10-1987 | MADDOX A.D. |